# EUROPEAN PATENT SPECIFICATION

(11) **EP 2 718 416 B1**
(45) Date of publication and mention of the grant of the patent: **13.11.2019**
(21) Application number: 12727636.8
(22) Date of filing: 06.06.2012
(51) Int. Cl.: C12M 1/12, C12M 3/06

(54) **EXPANSION OF STEM CELLS IN HOLLOW FIBER BIOREACTORS**
EXPANSION VON STAMMZELLEN IN HOHLFASERBIOREAKTOREN
MULTIPLICATION DE CELLULES SOUCHES DANS DES BIORÉACTEURS À FIBRES CREUSES

(30) Priority: 06.06.2011 US 201161493737 P
(43) Date of publication of application: 16.04.2014
(60) Divisional application: 19185835.6
(73) Proprietor: Regenesys bvba, 3001 Heverlee (BE)
(72) Inventor: PINXTEREN, Jozef, Albert, Martha, B-9030 Mariakerke (BE); CRAEYE, David, B-9200 Dendermonde (BE)
(74) Representative: J A Kemp LLP
(86) International application number: PCT/EP2012/060690
(87) International publication number: WO 2012/168295

(56) References cited:
- WO-A2-2008/109674
- WO-A2-2010/149597
- US-A1- 2008 248 572
- US-B1- 7 015 037
- "Bioreactor Design and Implementation" In: ANTWILER ET AL.: "Stem Cell Bioengineering", 2009 pages 49-62,
- ZHONG-BAORUAN: "Karyotype stability of human umbilical cord-derived mesenchymal stem cells during in vitro culture", EXPERIMENTAL AND THERAPEUTIC MEDICINE, vol. 8, 23 July 2014 (2014-07-23), pages 1508-1512, China
- Wikipedia: , 18 May 2017 (2017-05-18), Retrieved from the Internet: URL:https://en.wikipedia.org/wiki/Mesenchy mal_stem_cell [retrieved on 2017-05-18]
- NEDIME SERAKINCI, JESPER GRAAKJAER: "Telomerase stability and telomerase in mesenchymal stem cells", BIOCHIMIE, vol. 90, 25 September 2007 (2007-09-25), pages 33-40,

## Description

### FIELD OF THE INVENTION

The invention is directed to producing large numbers of cells using hollow fiber continuous perfusion bioreactor technology. The cells are non-embryonic stem, non-germ cells; express telomerase, are not transformed, have a normal karyotype, and can differentiate into at least one cell type of at least two of the endodermal, ectodermal, and mesodermal embryonic lineages.

### BACKGROUND OF THE INVENTION

Hollow fiber bioreactor technology has been used to obtain high density expansion of cells. Generally, cells are expanded in and/or outside of a multitude of hollow fibers. Because of the high surface area provided by this design, using the fibers as a culture substrate allows the production of large numbers of cells, especially for clinical applications. This technology, first developed in the 1970s by Knazek, has undergone numerous developments and improvements. The basic concept is providing a fiber matrix that is permeable to nutrients, gases, and other basic medium components, and to cellular waste products, but which is impermeable to the cells and upon which the cells can expand.

Cell culture on semi-permeable tubular membranes was initially invented by Knazek in the early 1970s. See, for example, U.S. 3,821,087; 3,883,393; 4,220,725; 4,184,922; and 4,200,689. In accordance with that invention, cells suspended in nutrient medium were allowed to settle on the outer surfaces of capillaries that were continuously perfused by oxygenated nutrient medium flowing through the capillaries. Nutrient substances diffused from the perfusion medium through the capillary wall and into the cells while cell waste products diffused from the cells through the capillary wall to the perfusate from which those products could be recovered.

That technology was developed as a response to failed attempts to grow cells to densities and/or structures approaching those of living tissue. Mainly, there were problems growing viable cells to very high densities such as to those that are found in tissues. First, components of the medium must diffuse through cell layers to reach all cells as the thickness of the cell layer increases. Second, a suitable micro-environment must be maintained during the cell culture. Thus, fluid immediately adjacent to the growing cells is continuously changing as cellular metabolism proceeds and is returned to its original state only in step-wise fashion when the culture medium is changed or agitated. Third, a lattice or suitable material on which to grow the dense cells is required. Knazek's invention solved these problems by providing nutrient sources within the cell mass that supply both large and small essential molecules; sinks within the cell mass remove the products of metabolism; a suitable micro-environment; a lattice to permit growth in three dimensions; and a surface area for mono- and/or multi-layer cell cultures that is large relative to the volume required by standard cell culture techniques.

Since the original invention, this technology has been successfully used to expand various cell types and there is large body of scientific and patent literature directed to various improvements of this basic technology. These improvements have dealt with various technical modifications to optimize the use of this technology to achieve the original goals of Knazek. In the industry, this technology has come to be designated "hollow fiber" cell culture and, in a bioreactor setting, provides a hollow fiber bioreactor cell culture. See, for example, U.S. Patents 3,997,396; 4,184,922; 4,200,689; 4,220,725; 4,804,628; 4,999,298; 5,126,238; 5,162,225; 5,627,070; 5,656,421; 5,712,154; 6,001,585; 6,680,166; 6,911,201; 6,933,144; 7,534,609; U.S. Publications 2001/0044413; 2003/0224510; 2005/0032218; 2005/0003530; 2006/0205071; 2007/0298497; 2008/0206733; 2008/0213894; 2008/0220522; 2008/0220523; 2008/0227190; 2008/248572; 2008/254533; 2009/0191631; 2009/0196901; 2010/0042260; 2010/0144037; 2010/0209403; 2010/0233130; 2010/0267134; WO 91/18972; WO 95/13088; WO 95/21911; WO 01/23520; WO 07/012144; WO 10/034468; WO 10/149597; and Gloeckner et al., Biotech Prog 17:828-31 (2001). For example, US 2008/248572 relates to treatment of polymeric bioreactor surfaces, to promote the proliferation of adherent cells.

U.S. 2010/0267134 discloses the isolation, characterization, and differentiation of adult human germ line stem cells using hollow fiber capillary culture. This reference describes growing spermatagonial stem cells on feeder layers of Sertoli cells that have been seeded onto the hollow fiber capillary surface.

U.S. 2007/0298497 and 2008/0220523 disclose hollow fiber bioreactor technology applied to mesenchymal stem cells. Cell expansion systems and methods of using them to grow mesenchymal stem cells are described.

U.S. 2009/0196901 relates to methods and systems for facilitating growth and differentiation of adipose-derived stem cells. The cells can be grown with a biologically-compatible scaffold structure that may comprise hollow fibers for cell growth and differentiation, the scaffold being cultured in a bioreactor for growth and differentiation of the cells. The reference suggests that other stem cell types may be used in this process. However, prior to growth and differentiation on the woven fiber scaffold for culture in the bioreactor, the cells have been expanded significantly in cell culture already. This produces a sufficient number of stem cells so that the cells can be concentrated and suspended in a matrix such as a gelbiomaterial and applied to the three-dimensional scaffold.

U.S. 2010/0209403 discloses methods for growing adherent placental and adipose mesenchymal stem cells in bioreactors that can comprise hollow fibers.

WO 95/13088 discloses hollow fiber technology for growing hematopoietic stem cells with stromal cell support.

WO 91/18972 discloses hollow fiber bioreactors for growing hematopoietic stem cells from bone marrow.

WO 07/012144 discloses hollow fiber bioreactors that are improved by attaching linkers to the surface to propagate embryonic stem cells and other cells.

WO 10/149597 discloses hollow fiber bioreactors for growing spermatagonial and other germ line cells.

WO 10/034468 discloses hollow fiber technology in which adherent cells are grown without a coating, for example, mesenchymal stem cells. WO 2008/109674 also discloses a cell expansion system for growing adherent cells, for example mesenchymal stem cells.

Antwiler et al.("Bioreactor Design and Implementation," Stem Cell Bioengineering, Parekkadan and Yarmush, eds., pp. 49-62 (2009)) discloses a method for the *ex vivo* expansion of mesenchymal stem cells from both bone marrow mononuclear cells and whole bone marrow using an automated hollow fiber bioreactor system. The system comprised a synthetic hollow fiber bioreactor connected to sterile closed-loop computer-controlled media and gas exchangers. In experimental settings, this allowed therapeutic doses of mesenchymal stem cells to be grown from small amounts of single bone marrow aspirates within a relatively short amount of time.

### SUMMARY OF THE INVENTION

The present invention relates to expanding the cells described herein in hollow fiber bioreactors. Continuous perfusion for continuous feed of nutrients and removal of waste metabolites is an aspect of the invention. Collection of desired secreted molecules is also an aspect.

The invention provides a method of expanding cells *ex vivo,* the method comprising:
a) seeding the cells on a hollow fiber substrate so that the cells adhere to the substrate;
b) expanding the adhered cells on the substrate; and
c) removing the expanded cells from the substrate;
wherein the cells are non-embryonic stem, non-germ cells; express telomerase, are not transformed, have a normal karyotype, and can differentiate into at least one cell type of at least two of the endodermal, ectodermal, and mesodermal embryonic lineages.

The cells may be expanded directly from a tissue sample, for example, from bone marrow, cord blood, or placenta, as unpurified or partially-purified preparations. Or the cells may be a purified preparation of cells that are substantially homogeneous, having been previously isolated and expanded or otherwise purified. Thus, the purity of the starting cell preparation may be virtually unpurified (such as starting with total bone marrow mononuclear cells) or from 1%-100% pure. In some embodiments the purity of the cells for administration to a subject is about 100% (substantially homogeneous). In other embodiments it is 95% to 100%. In some embodiments it is 85% to 95%. However, the percentage can be about 1%-5%, 5%-10%, 10%-15%, 15%-20%, 20%-25%, 25%-30%, 30%-35%, 35%-40%, 40%-45%, 45%-50%, 60%-70%, 70%- 80%, 80%-90%, or 90%-95%. Or isolation/purity can be expressed in terms of cell doublings where the cells have undergone, for example, 10-20, 20-30, 30-40, 40-50 or more cell doublings.

The cells may be derived from a single donor for expansion and return to that same donor (autologous). Or the cells may be derived from a single donor for expansion and administration to a different subject (allogeneic). Or the cells may be derived from different donors.

The cells may be expanded (proliferated) through any desired number of cell doublings. Ranges include from two-fold up to 1000-fold or more. The limit will depend on the capacity to provide sufficient nutrients and remove deleterious waste. In some embodiments, cell doubling is in the range of about 2- about 10 fold, about 10- about 50 fold, and about 50- about 100-fold. Using the technology described in this application, greater than 10⁸ cells can be produced from a single donor.

Cells may be expanded, harvested, and then re-introduced into the bioreactor for further expansion. For the purposes of this application, a "run" refers to one round of expansion (from introduction of the cells to harvesting). Accordingly, cells may be collected after one or more than one run. A "passage" refers to a run. Cells may be expanded through different cell doublings in each run. In a single run, for example, cells may expand two-tenfold. However, with repeated runs, the total expansion may be much higher (as discussed above).

The cells of the present disclosure may express pluripotency markers, such as oct4. They may also express markers associated with extended replicative capacity, such as telomerase. Other characteristics of pluripotency can include the ability to differentiate into cell types of more than one germ layer, such as two or three of ectodermal, endodermal, and mesodermal embryonic germ layers. Such cells may or may not be immortalized or transformed in culture. The cells may be highly expanded without being transformed and also maintain a normal karyotype. For example, in one embodiment, the non-embryonic stem, non-germ cells may have undergone at least 10-40 cell doublings in culture, such as 50, 60, or more, wherein the cells are not transformed and have a normal karyotype. The cells may differentiate into at least one cell type of each of two of the endodermal, ectodermal, and mesodermal embryonic lineages and may include differentiation into all three. Further, the cells may not be tumorigenic, such as not producing teratomas. If cells are transformed or tumorigenic, and it is desirable to use them for infusion, such cells may be disabled so they cannot form tumors *in vivo,* as by treatment that prevents cell proliferation into tumors. Such treatments are well known in the art.

Cells include, but are not limited to, the following numbered aspects of the disclosure:
1. Isolated expanded non-embryonic stem, non-germ cells, the cells having undergone at least 10-40 cell doublings in culture, wherein the cells express oct4, are not transformed, and have a normal karyotype.
2. The non-embryonic stem, non-germ cells of 1 above that further express one or more of telomerase, rex-1, rox-1, or sox-2.
3. The non-embryonic stem, non-germ cells of 1 above that can differentiate into at least one cell type of at least two of the endodermal, ectodermal, and mesodermal embryonic lineages.
4. The non-embryonic stem, non-germ cells of 3 above that further express one or more of telomerase, rex-1, rox-1, or sox-2.
5. The non-embryonic stem, non-germ cells of 3 above that can differentiate into at least one cell type of each of the endodermal, ectodermal, and mesodermal embryonic lineages.
6. The non-embryonic stem, non-germ cells of 5 above that further express one or more of telomerase, rex-1, rox-1, or sox-2.
7. Isolated expanded non-embryonic stem, non-germ cells that are obtained by culture of non-embryonic, non-germ tissue, the cells having undergone at least 40 cell doublings in culture, wherein the cells are not transformed and have a normal karyotype.
8. The non-embryonic stem, non-germ cells of 7 above that express one or more of oct4, telomerase, rex-1, rox-1, or sox-2.
9. The non-embryonic stem, non-germ cells of 7 above that can differentiate into at least one cell type of at least two of the endodermal, ectodermal, and mesodermal embryonic lineages.
10. The non-embryonic stem, non-germ cells of 9 above that express one or more of oct4, telomerase, rex-1, rox-1, or sox-2.
11. The non-embryonic stem, non-germ cells of 9 above that can differentiate into at least one cell type of each of the endodermal, ectodermal, and mesodermal embryonic lineages.
12. The non-embryonic stem, non-germ cells of 11 above that express one or more of oct4, telomerase, rex-1, rox-1, or sox-2.
13. Isolated expanded non-embryonic stem, non-germ cells, the cells having undergone at least 10-40 cell doublings in culture, wherein the cells express telomerase, are not transformed, and have a normal karyotype.
14. The non-embryonic stem, non-germ cells of 13 above that further express one or more of oct4, rex-1, rox-1, or sox-2.
15. The non-embryonic stem, non-germ cells of 13 above that can differentiate into at least one cell type of at least two of the endodermal, ectodermal, and mesodermal embryonic lineages.
16. The non-embryonic stem, non-germ cells of 15 above that further express one or more of oct4, rex-1, rox-1, or sox-2.
17. The non-embryonic stem, non-germ cells of 15 above that can differentiate into at least one cell type of each of the endodermal, ectodermal, and mesodermal embryonic lineages.
18. The non-embryonic stem, non-germ cells of 17 above that further express one or more of oct4, rex-1, rox-1, or sox-2.
19. Isolated expanded non-embryonic stem, non-germ cells that can differentiate into at least one cell type of at least two of the endodermal, ectodermal, and mesodermal embryonic lineages, said cells having undergone at least 10-40 cell doublings in culture.
20. The non-embryonic stem, non-germ cells of 19 above that express one or more of oct4, telomerase, rex-1, rox-1, or sox-2.
21. The non-embryonic stem, non-germ cells of 19 above that can differentiate into at least one cell type of each of the endodermal, ectodermal, and mesodermal embryonic lineages.
22. The non-embryonic stem, non-germ cells of 21 above that express one or more of oct4, telomerase, rex-1, rox-1, or sox-2.

The cells described above can be prepared from any desirable tissue source, including, but not limited to, bone marrow, umbilical cord blood, umbilical cord matrix, peripheral blood, placenta, placental blood, muscle, brain, kidney, and other solid organs. They can also be derived from excreted fluids, such as urine and menstrual blood.

In one embodiment, the cells are derived from human tissue.

In configuring the hollow fiber bioreactor, there are several design considerations and several parameters that can be varied depending upon the goals associated with expansion of the cells. First, the number of fibers in the bioreactor can be varied. A plurality or bundle of fibers is generally used to provide a desired surface area and a high density of cells. Practical ranges in the number of fibers will also vary depending upon the length of the fibers. Thus, the length is also variable. Generally, the length of the fibers is only limited by the ability to efficiently move nutrient medium into contact with the cells, remove waste or desired cellular products, and to effectively release the cells from the fiber substrate. Accordingly, the range in the length of the fibers can vary. A further design consideration is the thickness of the fiber wall. The wall thickness is variable and is limited only by the parameters of efficiently providing nutrients to the cells and efficiently removing waste or useful products from the cells. Another design consideration is the size of the pores in the fiber wall. This is generally designed to allow the passage of nutrients to the cells, carry away waste, provide desired products to the cells (such as growth factors), to remove desired products from the cells, and the like. For example, pore size can be designed to exclude certain factors that may be present, for example, in serum, from reaching the cells. A further design consideration is the inner dimension of the hollow fibers (such as the diameter of a round fiber). Considerations for this choice include adequate access of the culture medium to the cell, to provide adequate nutrients, remove waste, and to achieve desired cellular densities. A further design consideration is the composition of the fiber wall. This includes a wide variety of bio-compatible materials that provide a substrate on which cells can adhere. In some embodiments, adherence may be improved by coating the wall material with an adherence- promoting substrate, such as an extracellular matrix protein.

Furthermore, more than one type of fiber may be found in the bioreactor with respect to the various parameters described above. This is useful, for example, when it is desirable to co-culture different cell types. Optimal fiber design may differ for the different cells.

The plurality of hollow fibers is generally encased in an outer shell that is desirably connected to inlet and outlet ports for perfusing medium into and/or around the fibers. In one embodiment, these fibers are found as part of a growth chamber in which cell medium is perfused (circulated) so that medium flows in and/or around the fibers. In one embodiment, the fibers are placed in a cartridge that is connected to a pump for infusion of cell culture medium and supplements.

The materials of which the fibers can be made comprise a large variety of bio-compatible semi-permeable materials. Such materials permit cell growth in three dimensions while allowing nutrient medium to diffuse through the fiber walls to feed the cells.

The cells to which the invention is directed may be co-cultured with other cell types with which they are co-plated or which have been separately adhered or otherwise cultured in the expansion chamber. In one embodiment, the other cells may be attached to the interior (or exterior) surface of the fiber prior to introducing the cells of the invention. Accordingly, the cells of the invention may be deposited on a monolayer of a desirable cell type.

The hollow fibers can be pre-coated with one or more extracellular matrix proteins, for example, Matrigel, fibronectin, or collagen, to enhance cell attachment. Extracellular matrix proteins may be attached to the internal and/or external surface of the fibers. Generally, extracellular matrix protein may be attached to the surface by any of the methodologies as described in U.S. Pat. No. 5,872,094 and U.S. Pat. No. 6,471,689.

Flow rates are also variable depending upon the cells to be expanded and the stage of the process. For example, after achieving cell adhesion, after cell inoculation, the flow rate can be increased to a steady- state level. The rate would be increased after the adherence of the cells and again to facilitate the harvesting of the cells from within the chambers.

A custom-designed hollow-fiber capillary culture system could be manufactured using readily available materials for mammalian cell culture on a contractual basis by many different vendors. Some types of hollow-fiber systems for cell culture are commercially available from companies, for example, FiberCell Systems, Inc. (Frederick, Md.). The hollow fiber systems made by FiberCell Systems are composed of fibers that are approximately 200 microns in diameter. The fibers are sealed into a cartridge that is designed so that cell culture medium pumped through the end of the cartridge flows through the inside of the fiber. The cells are attached to a porous support and the cultures can be maintained for many months of continuous production.

Analysis of expanding/expanded cells may include expression of markers such as those described in this application. In addition, the lack of expression of hematopoietic markers such as CD34 and CD45 may be analyzed. The sample supernatant and monolayers can be periodically monitored for (a) cell morphology, (b) ploidy and (c) in situ hybridization for cell markers.

As indicated above, cells that are expanded in the bioreactor may first be isolated and cultured using other culture conditions. For example, the exemplified cells (designated "MAPC") may be first isolated and cultured as described herein below.

### BRIEF DESCRIPTION OF THE FIGURES

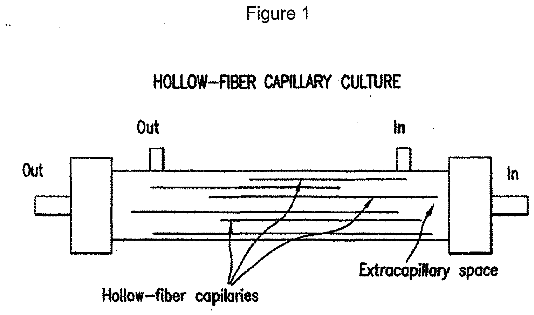

Figure 1 provides a schematic illustration of a hollow fiber cell expansion system.
Figure 2 is a schematic view of a bioreactor that may be used in this invention.
Figure 3A depicts a side view of a hollow fiber cell growth chamber embodiment of a cell growth chamber. Figure 3B depicts a cut-away side view of the hollow fiber cell growth chamber of the embodiment of Figure 3A.
Figure 4 - A cell expansion system (CES) fluid circuit showing block diagram system and all bag connections.
Figure 5 - Flow scheme for Quantum Cell Expansion System from Caridian BCT (now owned by Terumo) used to expand MultiStem from bone marrow or from cell banks.

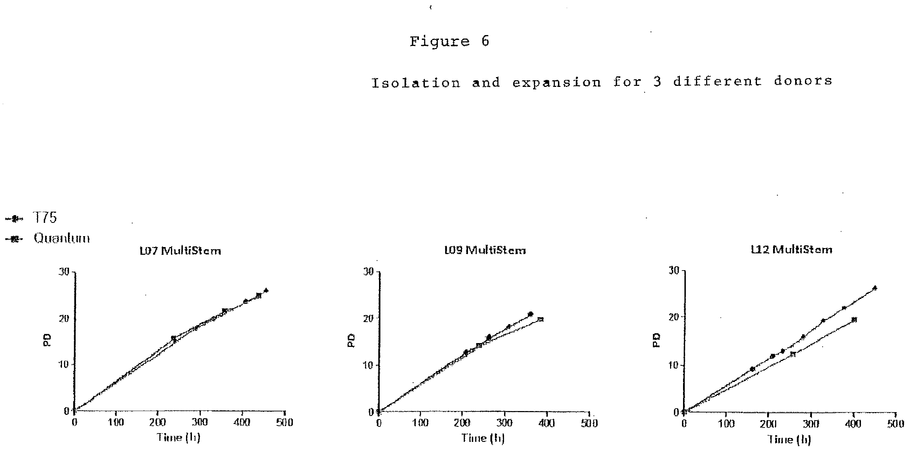

Figure 6 - A whole bone marrow aspirate was seeded in the Quantum without prior selection of the bone marrow mononuclear cells (BMMNC) and BMMNC from the same donor were seeded on regular cell culture plastic. This was done for 3 independent donors. Cells in the T75 were passaged at least 3 times and cell numbers were counted to calculate the population doubling (PD). Cells in the Quantum were harvested, counted, loaded onto a new bioreactor and harvested again 6 days later. Expansion rate for MultiStem starting from a bone marrow aspirate in the Quantum are not significantly different from expansion on cell culture plastic.
Figure 7 - Starting from a bone marrow aspirate, it is possible to create a MultiStem master cell bank within 17 days.

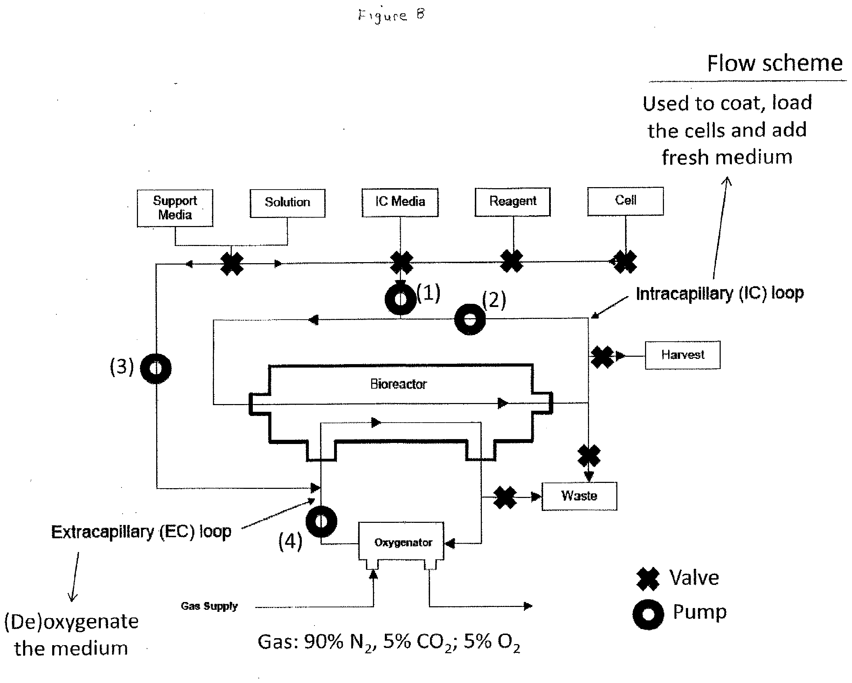

Figure 8 - More detailed scheme of apparatus of Figure 5.

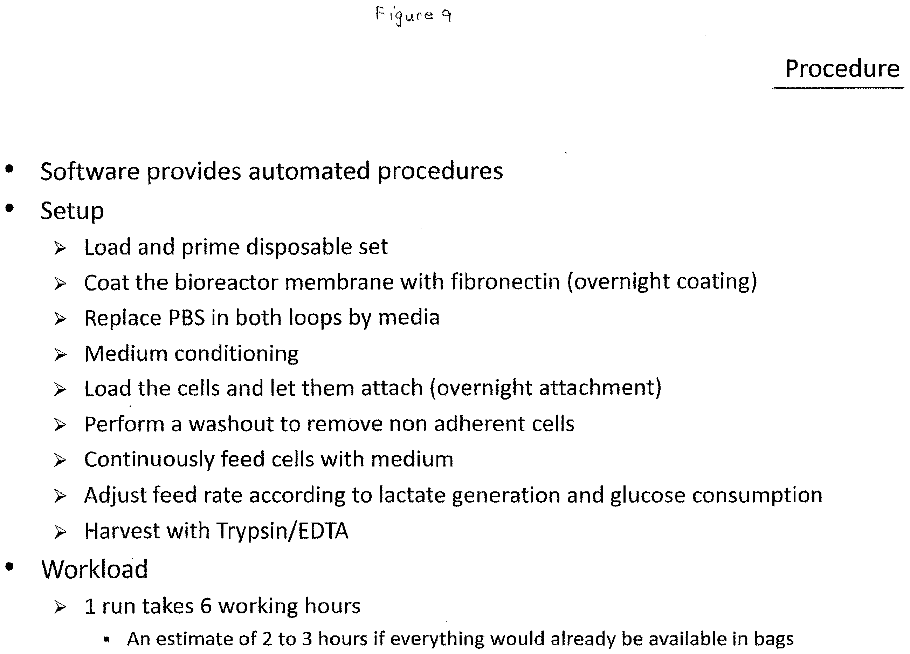

Figure 9 - Overview of an expansion embodiment with the closed system, continuous perfusion, hollow fiber bioreactor.

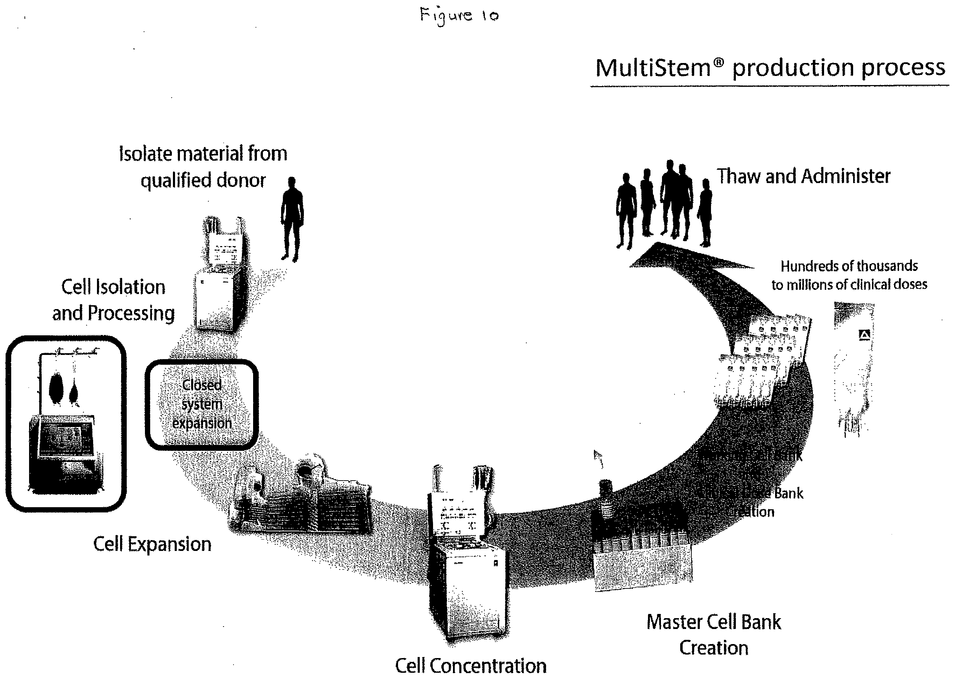

Figure 10 - Overview of the production process, from isolating the cells from a qualified donor to creating a master cell bank, and then administering the cells to a patient.

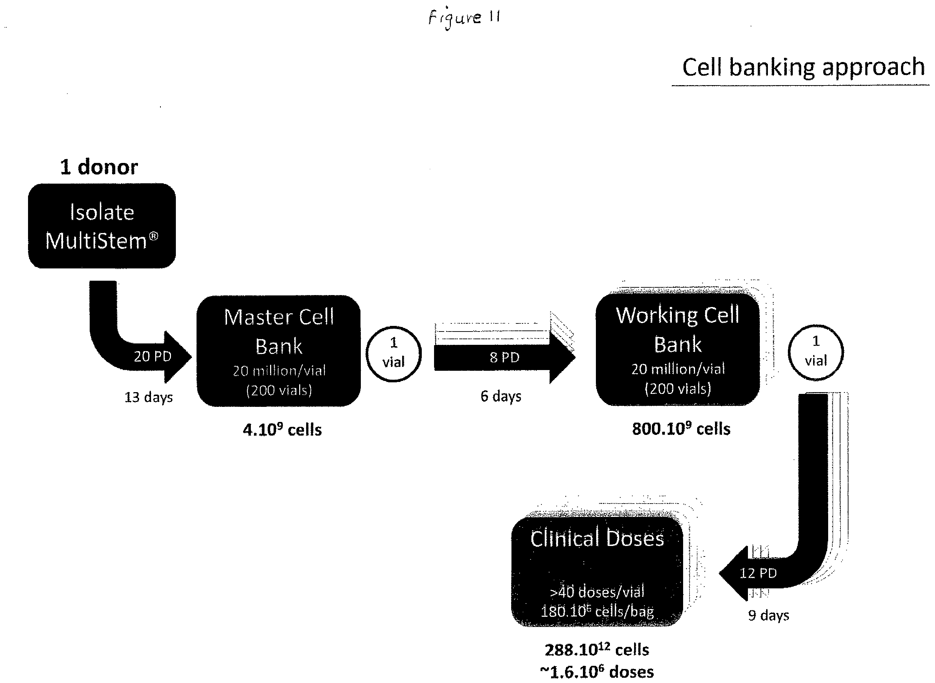

Figure 11 - Schematic of cell banking approach.

### DETAILED DESCRIPTION OF THE INVENTION

It should be understood that this invention is not limited to the particular methodology, protocols, and reagents, etc., described herein and, as such, may vary. The terminology used herein is for the purpose of describing particular embodiments only, and is not intended to limit the scope of the disclosed invention, which is defined solely by the claims.

The section headings are used herein for organizational purposes only and are not to be construed as in any way limiting the subject matter described.

The methods and techniques of the present application are generally performed according to conventional methods well-known in the art and as described in various general and more specific references that are cited and discussed throughout the present specification unless otherwise indicated. See, e.g., Sambrook et al., Molecular Cloning: A Laboratory Manual, 3rd ed., Cold Spring Harbor Laboratory Press, Cold Spring Harbor, N.Y. (2001) and Ausubel et al., Current Protocols in Molecular Biology, Greene Publishing Associates (1992), and Harlow and Lane, Antibodies: A Laboratory Manual, Cold Spring Harbor Laboratory Press, Cold Spring Harbor, N.Y. (1990).

### Definitions

"A" or "an" means herein one or more than one; at least one. Where the plural form is used herein, it generally includes the singular.

The term "bioreactor" refers to a cell culture system that provides nutrients to cells and removes metabolites, as well as furnishes a physio-chemical environment conducive to cell growth, in a closed sterile system.

As used herein, the term "bioreactor" refers to any device in which biological and/or biochemical processes develop under monitored and controlled environmental and operating conditions, for example, pH, temperature, pressure, nutrient supply and waste removal. According to the invention, the basic class of bioreactors suitable for use with the present invention includes hollow fiber bioreactors.

A "cell bank" is industry nomenclature for cells that have been grown and stored for future use. Cells may be stored in aliquots. They can be used directly out of storage or may be expanded after storage. This is a convenience so that there are "off the shelf cells available for administration. The cells may already be stored in a pharmaceutically-acceptable excipient so they may be directly administered or they may be mixed with an appropriate excipient when they are released from storage. Cells may be frozen or otherwise stored in a form to preserve viability. In one embodiment of the invention, cell banks are created in which the cells have been selected for enhanced modulation of activation of macrophages. Following release from storage, and prior to administration to the subject, it may be preferable to again assay the cells for potency, i.e., level of modulation of activation of macrophages. This can be done using any of the assays, direct or indirect, described in this application or otherwise known in the art. Then cells having the desired potency can then be administered to the subject for treatment. Banks can be made using cells derived from the individual to be treated (from their pre-natal tissues such as placenta, umbilical cord blood, or umbilical cord matrix or expanded from the individual at any time after birth) (autologous). Or banks can contain cells for allogeneic uses. A master cell bank is a reservoir of cells to provide an aliquot of cells that can be further expanded to provide doses for administration to a subject.

A "clinically-relevant" number of cells refers to a number of cells that is sufficient to effect a clinical response; that is, a prevention, reduction, amelioration, etc. of an undesirable pathological condition in a subject. A particular embodiment pertains to a number of cells that is sufficient to create a master cell bank.

" Co-administer" means to administer in conjunction with one another, together, coordinately, including simultaneous or sequential administration of two or more agents.

"Comprising" means, without other limitation, including the referent, necessarily, without any qualification or exclusion on what else may be included. For example, "a composition comprising x and y" encompasses any composition that contains x and y, no matter what other components may be present in the composition. Likewise, "a method comprising the step of x" encompasses any method in which x is carried out, whether x is the only step in the method or it is only one of the steps, no matter how many other steps there may be and no matter how simple or complex x is in comparison to them. "Comprised of and similar phrases using words of the root "comprise" are used herein as synonyms of "comprising" and have the same meaning.

"Comprised of' is a synonym of "comprising" (see above).

"Conditioned cell culture medium" is a term well-known in the art and refers to medium in which cells have been grown. Herein this means that the cells are grown for a sufficient time to secrete the factors that are effective to achieve any of the results described in this application.

Conditioned cell culture medium refers to medium in which cells have been cultured so as to secrete factors into the medium. For the purposes of the present invention, cells can be grown through a sufficient number of cell divisions so as to produce effective amounts of such factors so that the medium has the effects. Cells are removed from the medium by any of the known methods in the art, including, but not limited to, centrifugation, filtration, immunodepletion (e.g., via tagged antibodies and magnetic columns), and FACS sorting.

"EC cells" were discovered from analysis of a type of cancer called a teratocarcinoma. In 1964, researchers noted that a single cell in teratocarcinomas could be isolated and remain undifferentiated in culture. This type of stem cell became known as an embryonic carcinoma cell (EC cell).

"Effective amount" generally means an amount which provides the desired local or systemic effect. For example, an effective amount is an amount sufficient to effectuate a beneficial or desired clinical result. The effective amounts can be provided all at once in a single administration or in fractional amounts that provide the effective amount in several administrations. The precise determination of what would be considered an effective amount may be based on factors individual to each subject, including their size, age, injury, and/or disease or injury being treated, and amount of time since the injury occurred or the disease began. One skilled in the art will be able to determine the effective amount for a given subject based on these considerations which are routine in the art. As used herein, "effective dose" means the same as "effective amount."

"Effective route" generally means a route which provides for delivery of an agent to a desired compartment, system, or location. For example, an effective route is one through which an agent can be administered to provide at the desired site of action an amount of the agent sufficient to effectuate a beneficial or desired clinical result.

"Embryonic Stem Cells (ESC)" are well known in the art and have been prepared from many different mammalian species. Embryonic stem cells are stem cells derived from the inner cell mass of an early stage embryo known as a blastocyst. They are able to differentiate into all derivatives of the three primary germ layers: ectoderm, endoderm, and mesoderm. These include each of the more than 220 cell types in the adult body. The ES cells can become any tissue in the body, excluding placenta. Only the morula's cells are totipotent, able to become all tissues and a placenta. Some cells similar to ESCs may be produced by nuclear transfer of a somatic cell nucleus into an enucleated fertilized egg.

"Expand" means to achieve a clinically-relevant number of cell divisions of the output cell. "Clinically relevant" means that sufficient cell doublings of these cells are achieved in and/or on the fibers to provide clinically-relevant numbers of the output cells. Hollow fiber bioreactor technology has been used for purposes other than actual cell expansion that produces clinically-relevant numbers of output cells. For example, such bioreactors have been used to differentiate an input population of cells, to concentrate an input population of cells, to treat blood and other fluids by means of an input population of cells, and the like. In these embodiments, the cells associated with the fibers are not actually output cells. They are adhered to the fibers, not so that they will expand, but for a different purpose. Nevertheless, in the timeframes used to achieve these other purposes, some of the input cells may, in fact, expand. What distinguishes this from the current application of the technology is that the expansion of the cells is not achieved in clinically-relevant numbers. Cell doubling is limited.

The term "hollow fiber" is intended to include hollow structures (of any shape) containing pores of defined size, shape and density for use in delivering nutrients (in solution) to cells contained within a bioreactor and for removal of waste materials (in solution) from cells contained within a bioreactor. For purposes of the present invention, hollow fibers may be constructed of a resorbable or nonresorbable material. Fibers include, but are not limited to, tubular structures.

Use of the term "includes" is not intended to be limiting.

"Increase" or "increasing" means to induce entirely where there was no pre-existing presence or to increase the degree of.

"Induced pluripotent stem cells (IPSC or IPS cells)" are somatic cells that have been reprogrammed, for example, by introducing exogenous genes that confer on the somatic cell a less differentiated phenotype. These cells can then be induced to differentiate into less differentiated progeny. IPS cells have been derived using modifications of an approach originally discovered in 2006 (Yamanaka, S. et al., Cell Stem Cell, 1:39-49 (2007)). For example, in one instance, to create IPS cells, scientists started with skin cells that were then modified by a standard laboratory technique using retroviruses to insert genes into the cellular DNA. In one instance, the inserted genes were Oct4, Sox2, Lif4, and c-myc, known to act together as natural regulators to keep cells in an embryonic stem cell-like state. These cells have been described in the literature. See, for example, Wemig et al., PNAS, 105:5856-5861 (2008); Jaenisch et al., Cell, 132:567-582 (2008); Hanna et al., Cell, 133:250-264 (2008); and Brambrink et al., Cell Stem Cell, 2:151-159 (2008). These references teach IPSCs and methods for producing them. It is also possible that such cells can be created by specific culture conditions (exposure to specific agents).

The "input cell population" refers to the cell type introduced into the bioreactor for expansion of that cell type and that will ultimately form the output cell population. The input cell population can be introduced into the bioreactor in very small numbers. For example, in bone marrow, the desired input cell population may originally be found in cell numbers as low as one in a million. Alternatively, the input population may be substantially homogeneous, such as derived from a cell bank and further expanded in the bioreactor.

The term "isolated" refers to a cell or cells which are not associated with one or more cells or one or more cellular components that are associated with the cell or cells *in vivo.* An "enriched population" means a relative increase in numbers of a desired cell relative to one or more other cell types *in vivo* or in primary culture.

However, as used herein, the term "isolated" does not indicate the presence of only stem cells. Rather, the term "isolated" indicates that the cells are removed from their natural tissue environment and are present at a higher concentration as compared to the normal tissue environment. Accordingly, an "isolated" cell population may further include cell types in addition to stem cells and may include additional tissue components. This also can be expressed in terms of cell doublings, for example. A cell may have undergone 10, 20, 30, 40 or more doublings *in vitro* or *ex vivo* so that it is enriched compared to its original numbers *in vivo* or in its original tissue environment (e.g., bone marrow, peripheral blood, adipose tissue, etc.).

"MAPC" is an acronym for "multipotent adult progenitor cell." It refers to a cell that is not an embryonic stem cell or germ cell but has some characteristics of these. MAPC can be characterized in a number of alternative descriptions, each of which conferred novelty to the cells when they were discovered. They can, therefore, be characterized by one or more of those descriptions. First, they have extended replicative capacity in culture without being transformed (tumorigenic) and with a normal karyotype. Second, they may give rise to cell progeny of more than one germ layer, such as two or all three germ layers (i.e., endoderm, mesoderm and ectoderm) upon differentiation. Third, although they are not embryonic stem cells or germ cells, they may express markers of these primitive cell types so that MAPCs may express one or more of Oct 3/4 (i.e., Oct 3A), rex-1, and rox-1. They may also express one or more of sox-2 and SSEA-4. Fourth, like a stem cell, they may self-renew, that is, have an extended replication capacity without being transformed. This means that these cells express telomerase (i.e., have telomerase activity). Accordingly, the cell type that was designated "MAPC" may be characterized by alternative basic characteristics that describe the cell via some of its novel properties.

The term "adult" in MAPC is non-restrictive. It refers to a non-embryonic somatic cell. MAPCs are karyotypically normal and do not form teratomas *in vivo.* This acronym was first used in U.S. Patent No. 7,015,037 to describe a pluripotent cell isolated from bone marrow. However, cells with pluripotential markers and/or differentiation potential have been discovered subsequently and, for purposes of this invention, may be equivalent to those cells first designated "MAPC." Essential descriptions of the MAPC type of cell are provided in the Summary of the Invention above.

MAPC represents a more primitive progenitor cell population than MSC (Verfaillie, C.M., Trends Cell Biol 12:502-8 (2002), Jahagirdar, B.N., et al., Exp Hematol, 29:543-56 (2001); Reyes, M. and C.M. Verfaillie, Ann N YAcad Sci, 938:231-233 (2001); Jiang, Y. et al., Exp Hematol, 30896-904 (2002); and (Jiang, Y. et al., Nature, 418:41-9. (2002)).

The term "MultiStem®" is the trade name for a cell preparation based on the MAPCs of U.S. Patent No. 7,015,037, i.e., a non-embryonic stem, non-germ cell as described above. MultiStem® is prepared according to cell culture methods disclosed in this patent application, particularly, lower oxygen and higher serum. MultiStem® is highly expandable, karyotypically normal, and does not form teratomas *in vivo.* It may differentiate into cell lineages of more than one germ layer and may express one or more of telomerase, oct3/4, rex-1, rox-1, sox-2, and SSEA4.

The term "nutrient solution" is intended to include solutions entering a bioreactor and containing those nutrient materials essential to the culture of mammalian or vertebrate cells. Nutrient solutions may also contain additives that affect specific changes in phenotype of cells under culture or to contribute to changes in the matrix structure of the forming newly formed bone, such as, mineralization.

Nutrients are delivered to the cells in the bioreactor and may impact the growth and differentiation of cells contained in the bioreactor. The nutrient solutions are selected to provide sufficient nutrition to the cells to maintain viability, growth, and/or differentiation in the bioreactor. Those skilled in the art are capable of selecting an appropriate nutrient solution for the present invention. For example, media such as Dulbecco's modified Eagle's medium may be used and may be further supplemented with other suitable nutrients. Other suitable nutrients include fetal bovine serum, L-ascorbic acid,-2-phosphate, antibiotics, cell modulators such as dexamethasone, beta-glycerolphosphate, glucose, glutamine, amino acid supplements, inhibitors (or activators) of apoptosis such as glutathione-ethyl ester, antioxidants, caspase inhibitors, and cations and anions, e.g., magnesium, manganese, calcium, phosphate, chloride, sodium, potassium, zinc, and sulfate ions, and nitrates and nitrites. The remaining concentration of components in the nutrient solution should be sufficient to promote growth in the bioreactor and maintain viability of the cells.

The "output cell population" refers to the cells that one desires to harvest from the bioreactor after having been expanded in the bioreactor. Harvested cells are defined as those that are removed from the bioreactor that will then be used in the clinic or for other purposes, for example, research, clinical trials, and the like.

"Primordial embryonic germ cells" (PG or EG cells) can be cultured and stimulated to produce many less differentiated cell types.

"Progenitor cells" are cells produced during differentiation of a stem cell that have some, but not all, of the characteristics of their terminally-differentiated progeny. Defined progenitor cells, such as "cardiac progenitor cells," are committed to a lineage, but not to a specific or terminally differentiated cell type. The term "progenitor" as used in the acronym "MAPC" does not limit these cells to a particular lineage. A progenitor cell can form a progeny cell that is more highly differentiated than the progenitor cell.

Selection could be from cells in a tissue. For example, in this case, cells would be isolated from a desired tissue, expanded in culture, selected for a desired characteristic, and the selected cells further expanded.

"Self-renewal" refers to the ability to produce replicate daughter stem cells having differentiation potential that is identical to those from which they arose. A similar term used in this context is "proliferation."

"Serum-free medium" refers to medium in which serum is not present or, if present, is at levels at which the components of the serum have no effect on the growth or variability of the cells (i.e., are not actually necessary, such as residual or trace amounts).

"Stem cell" means a cell that can undergo self-renewal (i.e., progeny with the same differentiation potential) and also produce progeny cells that are more restricted in differentiation potential. Within the context of the invention, a stem cell would also encompass a more differentiated cell that has dedifferentiated, for example, by nuclear transfer, by fusion with a more primitive stem cell, by introduction of specific transcription factors, or by culture under specific conditions. See, for example, Wilmut et al., Nature, 385:810-813 (1997); Ying et al., Nature, 416:545-548 (2002); Guan et al., Nature, 440:1199-1203 (2006); Takahashi et al., Cell, 126:663-676 (2006); Okita et al., Nature, 448:313-317 (2007); and Takahashi et al., Cell, 131:861-872 (2007).

Dedifferentiation may also be caused by the administration of certain compounds or exposure to a physical environment *in vitro* or *in vivo* that would cause the dedifferentiation. Stem cells also may be derived from abnormal tissue, such as a teratocarcinoma and some other sources such as embryoid bodies (although these can be considered embryonic stem cells in that they are derived from embryonic tissue, although not directly from the inner cell mass). Stem cells may also be produced by introducing genes associated with stem cell function into a non-stem cell, such as an induced pluripotent stem cell.

"Subject" means a vertebrate, such as a mammal, such as a human. Mammals include, but are not limited to, humans, dogs, cats, horses, cows, and pigs.

The term "waste solution" is intended to include solutions exiting a bioreactor and containing waste byproducts of cellular metabolism. The concentrations of waste byproducts, for example ammonia, lactic acid, etc. and residual levels of nutrients such as glucose, in the waste solution can be used to assess the levels of metabolic activity of cells being cultured in a bioreactor.

### Bioreactors

Bioreactors, especially bioreactors used for tissue regeneration processes, are well known. See, for example, U.S. 6,306,169; 6,197,575; 6,080,581; 5,677,355; 5,433,909; 5,898,040.

A bioreactor is a generalized term that essentially covers any kind of vessel that is capable of incubating cells while providing a degree of protection for the cells' environment. A bioreactor may be a static vessel such as a flask or culture bag in which the variables (such as composition of growth media, oxygen concentration, pH levels, and osmolarity) are not fully controlled and monitored. On the other hand there are fully automated electromechanical state-of-the-art bioreactors in which all the variables are monitored and controllable. Many inter-combinations between these examples are well known to one of ordinary skill in cellular biotechnology.

Three different traditional approaches for the cultivation of isolated hematopoietic stem or progenitor cells have been described in the literature: the static, the stirred and the immobilized culture. Static cultivation takes place in very simple culture systems such well plates, tissue-culture flasks or gas- permeable culture bags. As the former two systems do not allow cell cultivation on a clinical scale, the latter is actually the most-often used technique for stem cell expansion (Purdy et al., J Hematother, 4:515-525 (1995); McNiece et al., Hematol Cell Ther, 4:82-86 (1999); and McNiece et al., ExpHematol, 28:1181-1186 (2000a)). All these systems have the advantage of being easy to handle, single-use devices, which enable an uncomplicated cell harvest. However, with all these systems, process control modulation is effected via control of the incubator environment, and there is no provision of continuous feeding. Thus, variations in culture conditions during cultivation (e.g., oxygen tension, pH, substrate, metabolite and cytokine concentrations) are critical factors in all three methods of static cultivation.

Stirred bioreactors are commonly used in animal cell culture, offering a homogenous environment, representative sampling, better access to process control and an increased oxygen transfer. Several of stirred techniques (spinner flasks and stirred vessel bioreactors) have been successfully implemented in the cultivation of hematopoietic cells (Zandstra et al., Biotechnol, 12:909-914 (1994)).

The immobilization of stem and progenitor cells is an attempt to reach local high cell densities and to imitate the three-dimensional structure of the tissue (such as bone marrow) without the use of stromal feeder layer. In immobilized biocatalyst reactors, the cells may be immobilized in or on a carrier, immobilized by linkage among one another to form larger particles or confined within membrane barriers. Most of the reactors can be run in a batch, fed-batch or continuous mode. Immobilized bioreactors are well known in the art, such as the conventional reactors such as Continuous Stirred Tank Reactors (CSTR) and Packed Bed Reactors (PBR) as described in standard text books such as Ullmann's Encyclopedia Of Industrial Chemistry: Fifth edition, T. Campbell, R. Pfefferkom and J. F. Rounsaville Eds, VCH Publishers 1985, Vol A4, pp 141-170; Ullmann's Encyclopedia Of Industrial Chemistry: Fifth ed., B. Elvers, S. Hawkins and G. Schulz Eds, VCH Publishers, 1992, Vol B4, pp 381-433; J. B. Butt "Reaction Kinetics And Reactor Design" Prentice-Hall, Inc., 1980, pp 185-241.

Thus, bioreactors can be grouped according to general categories including: static bioreactors, stirred flask bioreactors, rotating wall vessel bioreactors, hollow fiber bioreactors and direct perfusion bioreactors. Within the bioreactors, cells can be free, or immobilized, seeded on porous 3-dimensional scaffolds (hydrogel).

Hollow fiber bioreactors can be used to enhance the mass transfer during culture. Thus, for the present invention, the bioreactor is a hollow fiber bioreactor. Hollow fiber bioreactors may have the stem and/or progenitor cells embedded within the lumen of the fibers, with the medium perfusing the extra- lumenal space or, alternatively, may provide gas and medium perfusion through the hollow fibers, with the cells growing within the extralumenal space. Such hollow fiber bioreactors suitable for the present invention have been disclosed as described herein, and some are commercially available, such as the Caridian (Terumo) BCT Quantum Cell Expansion System.

A bioreactor is defined as any device that provides the physiological requirements of the cell (for example, pH, temperature, pressure, nutrients, supply and waste removal) for the standard production of cells and their products. A bioreactor can enhance mass transport in engineering bioreactors for cell proliferation. There is continuous nutrition of cells and removal of waste products. For autologous administration of cells, a bioreactor should be relatively compact, disposable, and economical. Generally, a large chamber/volume ratio is required. Finally, to keep cells in a specific differentiation state may require growth factors or serum components and, thus, a media-bearing bioreactor is desirable. These requirements are met by the hollow fiber bioreactor-based cell expansion system described in Antwiler et al.

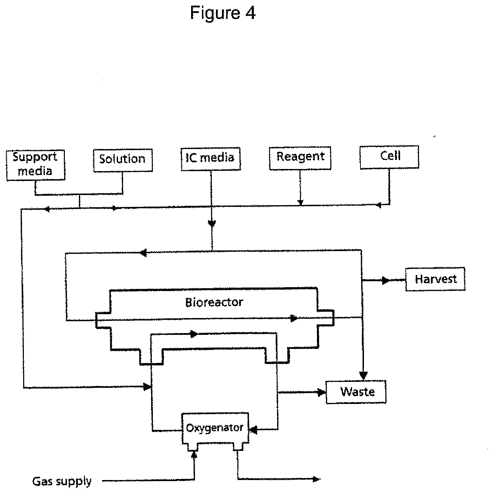

In the apparatus described in Antwiler et al., the system provides a chamber growth volume of around 120 ml although larger chamber volumes can be accommodated. For growth, a surface area of 1.7 m² is provided. Thus, both adherent and suspension cells can be grown in this device, including cocultures. The other physical characteristics are length, 295 mm; inner diameter, 215 µm; IC volume, 104 ml; EC volume, 330 ml; number of fibers, 9000. Fibronectin may be used as a coating for adherent cells. The device can maintain all required experimental conditions for growing a cell of interest, such as gas concentration, pH, media, temperature, management of nutrients, waste products, and required additives. To prevent contamination, the system is designed with a closed sterile fluid compartment. Bags of media reagents and solutions remain attached to the system so that feeding schedules, waste removal, and general cell culture environment can be replaced, controlled, and optimized as desired. The unit can be used on the bench top. Cells can be grown inside (intra-capillary), outside (extracapillary), or simultaneously on both sides of the fibers. This hollow fiber bioreactor design allows for direct connection using tubing which can be routed through appropriate peristaltic pumps, pinch valves, etc., to obtain a closed system. Gas control is managed using a hollow fiber oxygenator. The system also accommodates the ability to add and harvest cells, replace medium, and add reagents, etc. Thus, bags are used for all fluids and the bag connections all use sterile connecting technology. Schematic representation is found in Fig. 4.

In the exemplified embodiment in this application (Example 1) the continuous flow bioreactor has an internal fluid volume of 184 ml and an external fluid volume of 303 ml containing 1.1 x 10⁴ hollow fibers with a total surface area (intra-capillary) of approximately 2.1 m² (3255 in²), a wall thickness of 50 µm, inner-diameter of 215 µm, and length of 295 mm. Pore size has a 16 kDa cutoff for passage of components.

The hollow fibers should be suitable for the delivery of nutrients and removal of waste in the bioreactor. The hollow fibers may be any shape, for example, they may be round and tubular or in the form of concentric rings. The hollow fibers may be made up of a resorbable or non-resorbable membrane. For example, suitable components of the hollow fibers include polydioxanone, polylactide, polyglactin, polyglycolic acid, polylactic acid, polyglycolic acid/trimethylene carbonate, cellulose, methylcellulose, cellulosic polymers, cellulose ester, regenerated cellulose, pluronic, collagen, elastin, and mixtures thereof. The large variety of suitable materials is well-known in the art and is represented by a large body of literature. Examples of these materials have been described in U.S. Patents 4,220,725; 4,184,922; 4,200,689; 3,821,087; 3,883,393; 4,184,922; 4,200,689; 3,997,396; 4,220,725; 4,999,298; 4,804,628; 5,126,238; 5,656,421; 5,162,225; 5,622,857; 5,627,070; 6,001,585; 6,911,201; 6,933,144; 7,534,609; and U.S. Publications 2007/0298497; 2008/0220523; 2001/0044413; 2009/0196901; 2010/0233130; 2009/0191631; 2005/0032218; 2005/0003530; 2003/0224510; 2006/0205071; 2010/0267134; 2008/0206733; 2010/0209403, 2008/0213894; 2008/0220522; 2008/0227190; 2008/0248572; 2008/0254533; 2010/0144037; and 2010/0042260. The criteria for wall materials includes, but is not limited to, the following: not toxic to the cells; porous for the removal of waste and the reception of nutrients and, in instances where collection of components secreted by the cell is desired, porosity is adjusted for that parameter; relatively insensitive to temperature changes, i.e, thermally stable; able to retain shape integrity.

It is also known that such materials are impermeable to cells but permeable to a variety of desired materials. Thus, the hollow fibers include pores to allow the nutrients and waste to pass in and out of it. The pores of the hollow fibers are a sufficient diameter to allow the diffusion of a molecule from one side of the hollow fiber to the other side of the hollow fiber. Preferably, the molecules that may pass through the hollow fiber pores are about 0.002 to about 50 kDa, more preferably about 5 to about 25 kDa, or most preferably about 2 to about 16 kDa. Accordingly, the pore size of the fiber walls can be varied depending on the components that one desires to pass through the walls. For example, pore size can allow only small molecules or, maybe, larger to allow the passage of large proteinacious molecules, including growth factors, including, but not limited to, epidermal growth factor and platelet-derived growth factor. The person of ordinary skill in the art would understand how to vary the pore size depending upon the components that it is desirable to pass through the fiber walls to reach the cells or to carry material from the cells. For example, it may be desirable to collect components that are made by the cells for further use. The cells, for example, may express and secrete cytokines, growth factors, immunomodulatory factors, and other components that are further useful. Materials that may be carried from the cells also include waste products that one desires to remove from the cell. Accordingly, this technology could be used to produce medium conditioned by the cells containing useful components.

A number of bioreactor configurations exist for culturing anchorage-dependent cells, such as those of the invention. But this invention is not dependent on any particular configuration. One example is provided in U.S. Publication No. 2007/0298497, which discloses a configuration that has been used to grow mesenchymal stem cells.

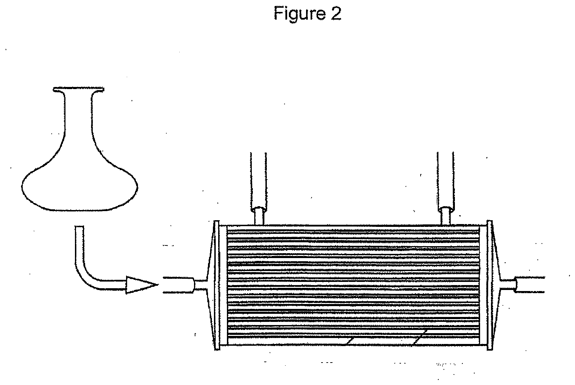

One example, in U.S. 2007/0298497 (not meant to be limiting), is a hollow fiber bioreactor shown in Figure 2. A cell expansion module or bioreactor 10 which may be used in the present invention is made of a bundle of hollow fiber membranes 12 enclosed within a housing 14. The bundle of hollow fibers is collectively referred to as a membrane. The housing or module 14 may be cylindrical in shape and may be made of any type of biocompatible polymeric material.

Each end of the module 14 is closed off with end caps or headers 16, 18. The end caps 16, 18 may be made of any suitable material such as polycarbonate so long as the material is biocompatible with the cells to be grown in the bioreactor.

There may be at least four ports into and out of the module. Two ports fluidly connect to the extracapillary space (EC space), one port 34 for fresh extracapillary media ingress into the space surrounding the hollow fibers and one port 44 for spent extracapillary media egress out of the module. Two ports also fluidly connect to the intracapillary space (IC space), one port 26 for fresh intracapillary media ingress into the lumen of the hollow fibers as well as for the introduction of the cells to be expanded, and one port 42 for spent intracapillary media egress and removal of expanded cells from the bioreactor.

Cells to be expanded in the bioreactor may be flowed into the IC space or the EC space. The bioreactor may be loaded with cells using a syringe or the cells may be distributed into the IC or EC spaces directly from a cell separator. The cells may also be introduced into the growth module or bioreactor from a cell input bag or flask (shown as element 5) which may be sterile docked to the bioreactor.

In one embodiment, the hollow fiber wall material that can be used includes, but is not limited to, two types, a 0.5% thermoplastic polyurethane sold under the trade name Desmopan.RTM. (available from Bayer MaterialScience AG, DE) and Polyflux.RTM., which is a trade name for a dialyzer with a membrane of Polyamix.RTM (a blend of polyamide, polyarylethersulfone and polyvinylpyrrolidone) (available from Gambro Dialysatoren, GmBH, Hechingen, Del.) (Hoenich, et al. (2000) ASAIO J., 46:70-75).

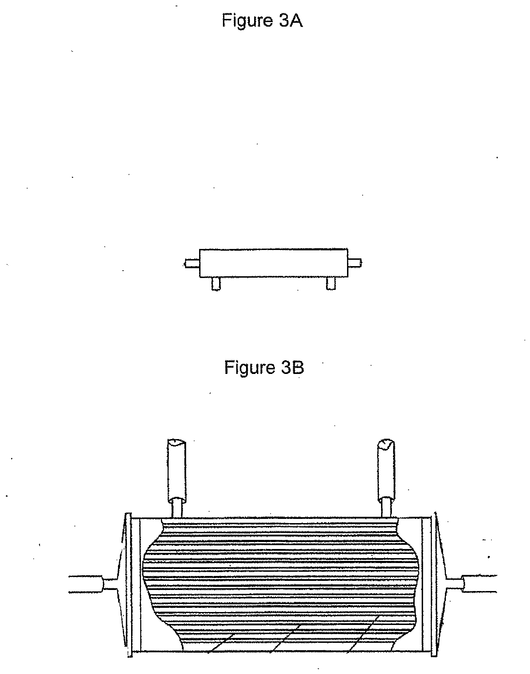

U.S. 2008/0220523 provides a cell expansion system and methods for using it that, in a specific embodiment, are suitable to grow the cells described herein. The cell expansion system generally includes a hollow fiber cell growth chamber and first and second circulation loops (intra-capillary loops and extracapillary loops) associated with the interior of the hollow fibers and exterior of the hollow fibers, respectively. Detachable flow circuits and methods of expanding cells are also provided. The cell growth chamber is described below and also is shown in Figure 3.

### Cell Growth Chamber (U.S. 2008/0220523)

The cell growth chamber of the cell expansion system generally includes a hollow fiber membrane comprised of a plurality of semi-permeable hollow fibers separating first and second fluid circulation paths.

An exemplary cell growth chamber is depicted in Figure 3, which depicts a cut-away side view of the hollow fiber cell growth chamber 200. Cell growth chamber 200 is bounded by cell growth chamber housing 202. Cell growth chamber housing 202 further includes four openings, or ports: inlet port 204, outlet port 206, inlet port 208, and outlet port 210.

Fluid in the first circulation path enters cell growth chamber 200 through inlet port 204, passes into and through the intracapillary side of a plurality of hollow fibers (referred to in various embodiments as the intracapillary ("IC") side or "IC space" of a hollow fiber membrane), and out of cell growth chamber 200 through outlet port 206. The terms "hollow fiber," "hollow fiber capillary," and "capillary" are used interchangeably. A plurality of hollow fibers is collectively referred to as a "membrane." Fluid in the second circulation path flows in the cell growth chamber through inlet port 208, comes in contact with the outside of the hollow fibers (referred to as the "EC side" or "EC space" of the membrane), and exits cell growth chamber 200 via outlet port 210. Cells can be contained within the first circulation path or second circulation path, and can be on either the IC side or EC side of the membrane.

Although cell growth chamber housing 202 is depicted as cylindrical in shape, it can have any other shape known in the art. Cell growth chamber housing 202 can be made of any type of biocompatible polymeric material. Various other cell growth chamber housings may differ in shape and size.

Those of skill in the art will recognize that the term "cell growth chamber" does not imply that all cells being grown or expanded in a cell expansion system are grown in the cell growth chamber. Adherent cells can adhere to membranes disposed in the growth chamber, or may grow within the associated tubing. Non-adherent cells (also referred to as "suspension cells") can also be grown. Cells can be grown in other areas within the first or second fluid circulation path.

For example, the ends of hollow fibers 212 can be potted to the sides of the cell growth chamber by a connective material (also referred to herein as "potting" or "potting material"). The potting can be any suitable material for binding the hollow fibers 212, provided that the flow of media and cells into the hollow fibers is not obstructed and that liquid flowing into the cell growth chamber through the IC inlet port flows only into the hollow fibers. Exemplary potting materials include, but are not limited to, polyurethane or other suitable binding or adhesive components. In various embodiments, the hollow fibers and potting may be cut through perpendicular to the central axis of the hollow fibers at each end to permit fluid flow into and out of the IC side. End caps 214 and 216 are disposed at the end of the cell growth chamber.

Fluid entering cell growth chamber 200 via inlet port 208 is in contact with the outside of hollow fibers. This portion of the hollow fiber cell growth chamber is referred to as the "extracapillary (EC) space." Small molecules (e.g. water, oxygen, lactate, etc.) can diffuse through the hollow fibers from the interior of the hollow fiber to the EC space, or from the EC space to the IC space. Large molecular weight molecules are typically too large to pass through the hollow fibers, and remain in the IC space of the hollow fibers. The media may be replaced as needed. Media may also be circulated through an oxygenator to exchange gasses as needed.

In various embodiments, cells can be loaded into the hollow fibers by any of a variety of methods, including by syringe. The cells may also be introduced into the cell growth chamber from a fluid container, such as a bag, which may be fluidly associated with the cell growth chamber.

Hollow fibers are configured to allow cells to grow in the intracapillary space (i.e. inside the hollow fiber lumen) of the fibers. Hollow fibers are large enough to allow cell adhesion in the lumen without substantially impeding the flow of media through the hollow fiber lumen. In various embodiments, the inner diameter of the hollow fiber can be greater than or equal to 10000, 9000, 8000, 7000, 6000, 5000, 4000, 3000, 2000, 1000, 900, 800, 700, 650, 600, 550, 500, 450, 400, 350, 300, 250, 200, 150, or 100 microns. Likewise, the outer diameter of the hollow fiber can be less than or equal to 10000, 9000, 8000, 7000, 6000, 5000, 4000, 3000, 2000, 1000, 900, 800, 700, 650, 700, 650, 600, 550, 500, 450, 400, 350, 300, 250, 200, 150, or 100 microns. The hollow fiber wall thickness is sufficient to allow diffusion of small molecules.

Any number of hollow fibers can be used in a cell growth chamber, provided the hollow fibers can be fluidly associated with the inlet and outlet ports of the cell growth chamber. In various embodiments, the cell growth chamber can include a number of hollow fibers greater than or equal to 1000, 2000, 3000, 4000, 5000, 6000, 7000, 8000, 9000, 10000, 11000 or 12000. In other embodiments, the cell growth chamber can include a number of hollow fibers less than or equal to 12000, 11000, 10000, 9000, 8000, 7000, 6000, 5000, 4000, 3000, or 2000. In other various embodiments, the length of the hollow fibers can be greater than or equal to 100, 200, 300, 400, 500, 600, 700, 800, or 900 millimeters. In certain embodiments, the cell growth chamber contains approximately 9000 hollow fibers that have an average length of 295 mm, an average inner diameter of 215 microns, and an average outer diameter of 315 microns.

Hollow fibers can be constructed of any material capable of forming a size sufficient to form fibers capable of transporting liquid from the cell growth chamber inlet port to the cell growth chamber outlet port. In various embodiments, the hollow fibers can be constructed from plastic adherent materials capable of binding to certain types of cells, such as adherent stem cells. In various other embodiments, hollow fibers can be treated with compounds such as fibronectin to form adherent surfaces.

In certain embodiments, the hollow fibers may be made of a semi-permeable, biocompatible polymeric material. One such polymeric material which can be used is a blend of polyamide, polyarylethersulfone and polyvinylpyrrolidone ("PA/PAES/PVP"). The semi-permeable membrane allows transfer of nutrients, waste and dissolved gases through the membrane between the EC space and IC space. In various embodiments, the molecular transfer characteristics of the hollow fiber membranes are chosen to minimize loss of expensive reagents necessary for cell growth such as growth factors, cytokines etc. from the hollow fiber, while allowing metabolic waste products to diffuse through the membrane into the hollow fiber lumen side to be removed.

In certain variations, one outer layer of each PA/PAES/PVP hollow fiber is characterized by a homogenous and open pore structure with a defined surface roughness. The openings of the pores are in the size range of 0.5-3 µm, and the number of pores on the outer surface of the fibers are in the range of 10,000 to 150,000 pores per mm². This outer layer has a thickness of about 1 to 10 µm. The next layer in each hollow fiber is a second layer having the form of a sponge structure and, in a further embodiment, a thickness of about 1 to 15 µm. This second layer serves as a support for the outer layer. A third layer next to the second layer has the form of finger-like structures. This third layer provides mechanical stability and a high void volume which gives the membrane a very low resistance to transporting molecules through the membrane. During use, the finger-like voids are filled with fluid and the fluid gives a lower resistance for diffusion and convection than a matrix with a sponge-filled structure having a lower void volume. This third layer has a thickness of 20 to 60 µm.

In further embodiments, the hollow fiber membrane can include 65-95% by weight of at least one hydrophobic polymer and 5-35% by weight of at least one hydrophilic polymer. The hydrophobic polymer may be chosen from the group consisting of polyamide (PA), polyaramide (PAA), polyarylethersulphone (PARS), polyethersulphone (PES), polysulphone (PSU), polyarylsulphone (PASU), polycarbonate (PC), polyether, polyurethane (PUR), polyetherimide and copolymer mixtures of any of the above polymers, such as polyethersulphone or a mix of polyarylethersulphone and polyamide. In additional embodiments, the hydrophilic polymer may be chosen from the group consisting of polyvinylpyrrolidone (PVP), polyethylene glycol (PEG), polyglycolmonoester, water soluble cellulosic derivates, polysorbate and polyethylene- polypropylene oxide copolymers.

Depending upon the type of cells to be expanded in the cell growth chamber, the polymeric fibers may be treated with a substance, such as fibronectin, to enhance cell growth and/or adherence of the cells to the membrane.

U.S. 2008/0220523 and U.S. 2008/0248572 also have examples of other detailed reactor embodiments, including detailed flow chart protocols. These various embodiments can be found, for example, in Figures IB, 1C, and 1D of U.S. 2008/0220523. The flow chart protocols pertain to Figure ID. Any of these would be suitable for practicing the invention. See also the embodiments disclosed in U.S. 2008/0220522.

WO/2010/034468 provides another exemplary device comprising two compartments separated by a semi-permeable membrane mounted in a casing, a first internal compartment fitted with two accesses and a second external compartment comprising one or two accesses, both compartments being also separated by a potting compound, based on an appropriate adhesive compound, intended for forming as applicable (i) a cylindrical partition separating both compartments of said device containing a semi-permeable membrane of the hollow fiber bundle type as defined above or (ii) a tight seal in said device including a semi-permeable membrane of the sheet membrane type as defined above.

Another exemplary device comprises a plurality of hollow fiber membranes, contained within an outer shell, and configured so that fluid within a space external to the hollow fibers (i.e., an extracapillary compartment) is segregated from fluid passing through the hollow fibers and their corresponding orifices. Additionally, the device includes two manifold end chambers within the outer shell on opposite ends of the device. Each of the two orifices of a hollow fiber connects to a different end chamber. The end chambers and the extracapillary compartment are separated by the semi-permeable membranes of the hollow fibers. The composition within the extracapillary compartment can be controlled, to a certain extent, by the molecular weight cutoff, or pore size, of the membranes of the hollow fibers.

In one mode of operating the device, cells are grown in the extracapillary compartment while a nutrient medium is passed through the hollow fibers. In another mode of operating the device, cells are grown in the intracapillary space (i.e. lumen) of the hollow fibers while a nutrient medium is passed through the extracapillary compartment and/or the intracapillary compartment. The semi-permeable nature of the hollow fibers allows nutrients and cell waste products to pass through the walls of the hollow fibers while blocking cells from doing the same.

Shell-and-tube type bioreactors provide several advantages. For adherent cells, the use of several hollow fibers provides, within a relatively small volume, a large amount of surface area upon which the cells can grow. This large amount of surface area also facilitates localized distribution of nutrient media to the growing cells and ready collection of cell waste products. Shell-and-tube type bioreactors enable the growth of cells at much higher density than is possible with other cell culture devices. They can support cell densities greater than 10⁸ cells per milliliter, whereas other cell culture devices are typically limited to densities around 10⁶ cells per milliliter.

### Stem Cells

The present invention can be practiced, preferably, using stem cells of vertebrate species, such as humans, non-human primates, domestic animals, livestock, and other non-human mammals. These include, but are not limited to, those cells described below.

### Embryonic Stem Cells

The most well studied stem cell is the embryonic stem cell (ESC) as it has unlimited self-renewal and multipotent differentiation potential. These cells are derived from the inner cell mass of the blastocyst or can be derived from the primordial germ cells of a post-implantation embryo (embryonal germ cells or EG cells). ES and EG cells have been derived, first from mouse, and later, from many different animals, and more recently, also from non-human primates and humans. When introduced into mouse blastocysts or blastocysts of other animals, ESCs can contribute to all tissues of the animal. ES and EG cells can be identified by positive staining with antibodies against SSEA1 (mouse) and SSEA4 (human). See, for example, U.S. Patent Nos. 5,453,357; 5,656,479; 5,670,372; 5,843,780; 5,874,301; 5,914,268; 6,110,739 6,190,910; 6,200,806; 6,432,711; 6,436,701, 6,500,668; 6,703,279; 6,875,607; 7,029,913; 7,112,437; 7,145,057; 7,153,684; and 7,294,508, each of which teach embryonic stem cells and methods of making and expanding them. Accordingly, ESCs and methods for isolating and expanding them are well-known in the art.

A number of transcription factors and exogenous cytokines have been identified that influence the potency status of embryonic stem cells *in vivo.* The first transcription factor to be described that is involved in stem cell pluripotency is Oct4. Oct4 belongs to the POU (Pit-Oct-Unc) family of transcription factors and is a DNA binding protein that is able to activate the transcription of genes, containing an octameric sequence called "the octamer motif within the promoter or enhancer region. Oct4 is expressed at the moment of the cleavage stage of the fertilized zygote until the egg cylinder is formed. The function of Oct3/4 is to repress differentiation inducing genes (i.e., FoxaD3, hCG) and to activate genes promoting pluripotency (FGF4, Utfl, Rexl). Sox2, a member of the high mobility group (HMG) box transcription factors, cooperates with Oct4 to activate transcription of genes expressed in the inner cell mass. It is essential that Oct3/4 expression in embryonic stem cells is maintained between certain levels. Overexpression or downregulation of >50% of Oct4 expression level will alter embryonic stem cell fate, with the formation of primitive endoderm/mesoderm or trophectoderm, respectively. *In vivo,* Oct4 deficient embryos develop to the blastocyst stage, but the inner cell mass cells are not pluripotent. Instead they differentiate along the extraembryonic trophoblast lineage. Sall4, a mammalian Spalt transcription factor, is an upstream regulator of Oct4, and is therefore important to maintain appropriate levels of Oct4 during early phases of embryology. When Sall4 levels fall below a certain threshold, trophectodermal cells will expand ectopically into the inner cell mass. Another transcription factor required for pluripotency is Nanog, named after a celtic tribe "Tir Nan Og": the land of the ever young. *In vivo,* Nanog is expressed from the stage of the compacted morula, is subsequently defined to the inner cell mass, and is down-regulated by the implantation stage. Downregulation of Nanog may be important to avoid an uncontrolled expansion of pluripotent cells and to allow multilineage differentiation during gastrulation. Nanog null embryos, isolated at day 5.5, consist of a disorganized blastocyst, mainly containing extraembryonic endoderm and no discernable epiblast.

### Non-Embrvonic Stem Cells

Stem cells have been identified in most tissues. Perhaps the best characterized is the hematopoietic stem cell (HSC). HSCs are mesoderm-derived cells that can be purified using cell surface markers and functional characteristics. They have been isolated from bone marrow, peripheral blood, cord blood, fetal liver, and yolk sac. They initiate hematopoiesis and generate multiple hematopoietic lineages. When transplanted into lethally-irradiated animals, they can repopulate the erythroid neutrophil-macrophage, megakaryocyte, and lymphoid hematopoietic cell pool. They can also be induced to undergo some self-renewal cell division. See, for example, U.S. Patent Nos. 5,635,387; 5,460,964; 5,677,136; 5,750,397; 5,681,599; and 5,716,827. U.S. Patent No. 5,192,553 reports methods for isolating human neonatal or fetal hematopoietic stem or progenitor cells. U.S. Patent No. 5,716,827 reports human hematopoietic cells that are Thy-1⁺ progenitors, and appropriate growth media to regenerate them *in vitro.* U.S. Patent No. 5,635,387 reports a method and device for culturing human hematopoietic cells and their precursors. U.S. Patent No. 6,015,554 describes a method of reconstituting human lymphoid and dendritic cells. Accordingly, HSCs and methods for isolating and expanding them are well-known in the art.

Another stem cell that is well-known in the art is the neural stem cell (NSC). These cells can proliferate *in vivo* and continuously regenerate at least some neuronal cells. When cultured ex vivo, neural stem cells can be induced to proliferate as well as differentiate into different types of neurons and glial cells. When transplanted into the brain, neural stem cells can engraft and generate neural and glial cells. See, for example, Gage F.H., Science, 287:1433-1438 (2000), Svendsen S.N. et al, Brain Pathology, 9:499-513 (1999), and Okabe S. et al., Mech Development, 59:89-102 (1996). U.S. 5,851,832 reports multipotent neural stem cells obtained from brain tissue. U.S. 5,766,948 reports producing neuroblasts from newborn cerebral hemispheres. U.S. 5,564,183 and 5,849,553 report the use of mammalian neural crest stem cells. U.S. 6,040,180 reports *in vitro* generation of differentiated neurons from cultures of mammalian multipotential CNS stem cells. WO 98/50526 and WO 99/01159 report generation and isolation of neuroepithelial stem cells, oligodendrocyte-astrocyte precursors, and lineage-restricted neuronal precursors. U.S. 5,968,829 reports neural stem cells obtained from embryonic forebrain. Accordingly, neural stem cells and methods for making and expanding them are well-known in the art.

Another stem cell that has been studied extensively in the art is the mesenchymal stem cell (MSC). MSCs are derived from the embryonal mesoderm and can be isolated from many sources, including adult bone marrow, peripheral blood, fat, placenta, and umbilical blood, among others. MSCs can differentiate into many mesodermal tissues, including muscle, bone, cartilage, fat, and tendon. There is considerable literature on these cells. See, for example, U.S. 5,486,389; 5,827,735; 5,811,094; 5,736,396; 5,837,539; 5,837,670; and 5,827,740. See also Pittenger, M. et al, Science, 284:143-147 (1999).

Another example of an adult stem cell is adipose-derived adult stem cells (ADSCs) which have been isolated from fat, typically by liposuction followed by release of the ADSCs using collagenase. ADSCs are similar in many ways to MSCs derived from bone marrow, except that it is possible to isolate many more cells from fat. These cells have been reported to differentiate into bone, fat, muscle, cartilage, and neurons. A method of isolation has been described in U.S. 2005/0153442.

Other stem cells that are known in the art include gastrointestinal stem cells, epidermal stem cells, and hepatic stem cells, which have also been termed "oval cells" (Potten, C., et al., Trans R Soc Lond B Biol Sci, 353:821-830 (1998), Watt, F., Trans R Soc Lond B Biol Sci, 353:831 (1997); Alison et al., Hepatology, 29:678-683 (1998).

Other non-embryonic cells reported to be capable of differentiating into cell types of more than one embryonic germ layer include, but are not limited to, cells from umbilical cord blood (see U.S. Publication No. 2002/0164794), placenta (see U.S. Publication No. 2003/0181269, umbilical cord matrix (Mitchell, K.E. et al., Stem Cells, 21:50-60 (2003)), small embryonic-like stem cells (Kucia, M. et al., J Physiol Pharmacol, 57 Suppl 5:5-18 (2006)), amniotic fluid stem cells (Atala, A., J Tissue Regen Med, 1:83- 96 (2007)), skin-derived precursors (Toma et al., Nat Cell Biol, 3:778-784 (2001)), and bone marrow (see U.S. Publication Nos. 2003/0059414 and 2006/0147246).

### Strategies of Reprogramming Somatic Cells

Several different strategies such as nuclear transplantation, cellular fusion, and culture induced reprogramming have been employed to induce the conversion of differentiated cells into an embryonic state. Nuclear transfer involves the injection of a somatic nucleus into an enucleated oocyte, which, upon transfer into a surrogate mother, can give rise to a clone ("reproductive cloning"), or, upon explantation in culture, can give rise to genetically matched embryonic stem (ES) cells ("somatic cell nuclear transfer," SCNT). Cell fusion of somatic cells with ES cells results in the generation of hybrids that show all features of pluripotent ES cells. Explantation of somatic cells in culture selects for immortal cell lines that may be pluripotent or multipotent. At present, spermatogonial stem cells are the only source of pluripotent cells that can be derived from postnatal animals. Transduction of somatic cells with defined factors can initiate reprogramming to a pluripotent state. These experimental approaches have been extensively reviewed (Hochedlinger and Jaenisch, Nature, 441:1061-1067 (2006) and Yamanaka, S., Cell Stem Cell, 1:39-49 (2007)).

### Nuclear Transfer

Nuclear transplantation (NT), also referred to as somatic cell nuclear transfer (SCNT), denotes the introduction of a nucleus from a donor somatic cell into an enucleated ogocyte to generate a cloned animal such as Dolly the sheep (Wilmut et al., Nature, 385:810-813 (1997). The generation of live animals by NT demonstrated that the epigenetic state of somatic cells, including that of terminally differentiated cells, while stable, is not irreversible fixed but can be reprogrammed to an embryonic state that is capable of directing development of a new organism. In addition to providing an exciting experimental approach for elucidating the basic epigenetic mechanisms involved in embryonic development and disease, nuclear cloning technology is of potential interest for patient-specific transplantation medicine.

### Fusion of Somatic Cells and Embryonic Stem Cells

Epigenetic reprogramming of somatic nuclei to an undifferentiated state has been demonstrated in murine hybrids produced by fusion of embryonic cells with somatic cells. Hybrids between various somatic cells and embryonic carcinoma cells (Solter, D., Nat Rev Genet, 7:319-327 (2006), embryonic germ (EG), or ES cells (Zwaka and Thomson, Development, 132:227-233 (2005)) share many features with the parental embryonic cells, indicating that the pluripotent phenotype is dominant in such fusion products. As with mouse (Tada et al, Curr Biol, 11:1553-1558 (2001)), human ES cells have the potential to reprogram somatic nuclei after fusion (Cowan et al, Science, 309:1369-1373(2005)); Yu et al, Science, 318:1917-1920 (2006)). Activation of silent pluripotency markers such as Oct4 or reactivation of the inactive somatic X chromosome provided molecular evidence for reprogramming of the somatic genome in the hybrid cells. It has been suggested that DNA replication is essential for the activation of pluripotency markers, which is first observed 2 days after fusion (Do and Scholer, Stem Cells, 22:941-949 (2004)), and that forced overexpression of Nanog in ES cells promotes pluripotency when fused with neural stem cells (Silva et al., Nature, 441:997-1001 (2006)).

### Culture-Induced Reprogramming

Pluripotent cells have been derived from embryonic sources such as blastomeres and the inner cell mass (ICM) of the blastocyst (ES cells), the epiblast (EpiSC cells), primordial germ cells (EG cells), and postnatal spermatogonial stem cells ("maGSCsm" "ES-like" cells). The following pluripotent cells, along with their donor cell/tissue is as follows: parthogenetic ES cells are derived from murine oocytes (Narasimha et al., Curr Biol, 7:881-884 (1997)); embryonic stem cells have been derived from blastomeres (Wakayama et al., Stem Cells, 25:986-993 (2007)); inner cell mass cells (source not applicable) (Eggan et al., Nature, 428:44-49 (2004)); embryonic germ and embryonal carcinoma cells have been derived from primordial germ cells (Matsui et al., Cell, 70:841-847 (1992)); GMCS, maSSC, and MASC have been derived from spermatogonial stem cells (Guan et al., Nature, 440:1199-1203 (2006); Kanatsu-Shinohara et al., Cell, 119:1001-1012 (2004); and Seandel et al., Nature, 449:346-350 (2007)); EpiSC cells are derived from epiblasts (Brons et al., Nature, 448:191-195 (2007); Tesar et al., Nature, 448:196-199(2007)); parthogenetic ES cells have been derived from human oocytes (Cibelli et al., Science, 295L819 (2002); Revazova et al., Cloning Stem Cells, 9:432-449 (2007)); human ES cells have been derived from human blastocysts (Thomson et al., Science, 282:1145-1147 (1998)); MAPC have been derived from bone marrow (Jiang et al.. Nature, 418:41-49 (2002); Phinney and Prockop, Stem Cells, 25:2896-2902 (2007)); cord blood cells (derived from cord blood) (van de Ven et al., Exp Hematol, 35:1753-1765 (2007)); neurosphere derived cells derived from neural cell (Clarke et al., Science, 288:1660-1663 (2000)). Donor cells from the germ cell lineage such as PGCs or spermatogonial stem cells are known to be unipotent *in vivo,* but it has been shown that pluripotent ES-like cells (Kanatsu-Shinohara et al., Cell, 119:1001-1012 (2004) or maGSCs (Guan et al., Nature, 440:1199-1203 (2006), can be isolated after prolonged *in vitro* culture. While most of these pluripotent cell types were capable of in vitro differentiation and teratoma formation, only ES, EG, EC, and the spermatogonial stem cell-derived maGCSs or ES-like cells were pluripotent by more stringent criteria, as they were able to form postnatal chimeras and contribute to the germline. Recently, multipotent adult spermatogonial stem cells (MASCs) were derived from testicular spermatogonial stem cells of adult mice, and these cells had an expression profile different from that of ES cells (Seandel et al, Nature, 449:346-350 (2007)) but similar to EpiSC cells, which were derived from the epiblast of postimplantation mouse embryos (Brons et al, Nature, 448:191-195 (2007); Tesar et al, Nature, 448:196-199 (2007)).

### Reprogramming by Defined Transcription Factors

Takahashi and Yamanaka have reported reprogramming somatic cells back to an ES-like state (Takahashi and Yamanaka, Cell, 126:663-676 (2006)). They successfully reprogrammed mouse embryonic fibroblasts (MEFs) and adult fibroblasts to pluripotent ES-like cells after viral-mediated transduction of the four transcription factors Oct4, Sox2, c-myc, and Klf4 followed by selection for activation of the Oct4 target gene Fbx15 (Figure 2A). Cells that had activated Fbxl5 were coined iPS (induced pluripotent stem) cells and were shown to be pluripotent by their ability to form teratomas, although the were unable to generate live chimeras. This pluripotent state was dependent on the continuous viral expression of the transduced Oct4 and Sox2 genes, whereas the endogenous Oct4 and Nanog genes were either not expressed or were expressed at a lower level than in ES cells, and their respective promoters were found to be largely methylated. This is consistent with the conclusion that the Fbxl5-iPS cells did not correspond to ES cells but may have represented an incomplete state of reprogramming. While genetic experiments had established that Oct4 and Sox2 are essential for pluripotency (Chambers and Smith, Oncogene, 23:7150-7160 (2004); Ivanona et al., Nature, 442:5330538 (2006); Masui et al., Nat Cell Biol, 9:625-635 (2007)), the role of the two oncogenes c-myc and Klf4 in reprogramming is less clear. Some of these oncogenes may, in fact, be dispensable for reprogramming, as both mouse and human iPS cells have been obtained in the absence of c- myc transduction, although with low efficiency (Nakagawa et al., Nat Biotechnol, 26:191-106 (2008); Weming et al., Nature, 448:318-324 (2008); Yu et al., Science, 318: 1917-1920 (2007)).

### MAPC

Human MAPCs are described in U.S. Patent 7,015,037. MAPCs have been identified in other mammals. Murine MAPCs, for example, are also described in U.S. Patent 7,015,037. Rat MAPCs are also described in U.S. Patent No. 7,838,289.

These references describe MAPCs first isolated by Catherine Verfaillie.

### Isolation and Growth of MAPCs

Methods of MAPC isolation are known in the art. See, for example, U.S. Patent 7,015,037, and these methods, along with the characterization (phenotype) of MAPCs. MAPCs can be isolated from multiple sources, including, but not limited to, bone marrow, placenta, umbilical cord and cord blood, muscle, brain, liver, spinal cord, blood or skin. It is, therefore, possible to obtain bone marrow aspirates, brain or liver biopsies, and other organs, and isolate the cells using positive or negative selection techniques available to those of skill in the art, relying upon the genes that are expressed (or not expressed) in these cells (e.g., by functional or morphological assays such as those disclosed in the above-referenced applications).

MAPCs have also been obtained by modified methods described in Breyer et al., Experimental Hematology, 34:1596-1601 (2006) and Subramanian et al., Cellular Programming and Reprogramming: Methods and Protocols; S. Ding (ed.), Methods in Molecular Biology, 636:55-78 (2010).

### MAPCs from Human Bone Marrow as Described in U.S. Patent 7,015,037

MAPCs do not express the common leukocyte antigen CD45 or erythroblast specific glycophorin-A (Gly-A). The mixed population of cells was subjected to a Ficoll Hypaque separation. The cells were then subjected to negative selection using anti-CD45 and anti-Gly-A antibodies, depleting the population of CD45⁺ and Gly-A⁺ cells, and the remaining approximately 0.1% of marrow mononuclear cells were then recovered. Cells could also be plated in fibronectin-coated wells and cultured as described below for 2-4 weeks to deplete the cells of CD45⁺ and Gly-A⁺ cells. In cultures of adherent bone marrow cells, many adherent stromal cells undergo replicative senescence around cell doubling 30 and a more homogenous population of cells continues to expand and maintains long telomeres.

Alternatively, positive selection could be used to isolate cells via a combination of cell-specific markers. Both positive and negative selection techniques are available to those of skill in the art, and numerous monoclonal and polyclonal antibodies suitable for negative selection purposes are also available in the art (see, for example, Leukocyte Typing V, Schlossman, et al., Eds. (1995) Oxford University Press) and are commercially available from a number of sources.

Techniques for mammalian cell separation from a mixture of cell populations have also been described by Schwartz, et al., in U. S. Patent No. 5,759,793 (magnetic separation), Basch et al., 1983 (immunoaffinity chromatography), and Wysocki and Sato, 1978 (fluorescence-activated cell sorting).

Cells may be cultured in low-serum or serum-free culture medium. Serum-free medium used to culture MAPCs is described in U.S. Patent 7,015,037. Commonly-used growth factors include but are not limited to platelet-derived growth factor and epidermal growth factor. See, for example, U.S. Patent Nos. 7,169,610; 7,109,032; 7,037,721; 6,617,161; 6,617,159; 6,372,210; 6,224,860; 6,037,174; 5,908,782; 5,766,951; 5,397,706; and 4,657,866; all teach growing cells in serum-free medium.

### Additional Culture Methods

In additional experiments the density at which MAPCs are cultured can vary from about 100 cells/cm² or about 150 cells/cm² to about 10,000 cells/cm², including about 200 cells/cm² to about 1500 cells/cm² to about 2000 cells/cm². The density can vary between species. Additionally, optimal density can vary depending on culture conditions and source of cells. It is within the skill of the ordinary artisan to determine the optimal density for a given set of culture conditions and cells.

Also, effective atmospheric oxygen concentrations of less than about 10%, including about 1-5% and, especially, 3-5%, can be used at any time during the isolation, growth and differentiation of MAPCs in culture.

Cells may be cultured under various serum concentrations, e.g., about 2-20%. Fetal bovine serum may be used. Higher serum may be used in combination with lower oxygen tensions, for example, about 15-20%. Cells need not be selected prior to adherence to culture dishes. For example, after a Ficoll gradient, cells can be directly plated, e.g., 250,000-500,000/cm². Adherent colonies can be picked, possibly pooled, and expanded.

In one embodiment, used in the experimental procedures in the Examples, high serum (around 15- 20%) and low oxygen (around 3-5%) conditions were used for the cell culture. Specifically, adherent cells from colonies were plated and passaged at densities of about 1700-2300 cells/cm² in 18% serum and 3% oxygen (with PDGF and EGF).

In an embodiment specific for MAPCs, supplements are cellular factors or components that allow MAPCs to retain the ability to differentiate into cell types of more than one embryonic lineage, such as all three lineages. This may be indicated by the expression of specific markers of the undifferentiated state, such as Oct 3/4 (Oct 3A) and/or markers of high expansion capacity, such as telomerase.

### Cell Culture

For all the components listed below, see U.S. 7,015,037, which teaches these components.

In general, cells useful for the invention can be maintained and expanded in culture medium that is available and well-known in the art. Also contemplated is supplementation of cell culture medium with mammalian sera. Additional supplements can also be used advantageously to supply the cells with the necessary trace elements for optimal growth and expansion. Hormones can also be advantageously used in cell culture. Lipids and lipid carriers can also be used to supplement cell culture media, depending on the type of cell and the fate of the differentiated cell. Also contemplated is the use of feeder cell layers.

Stem cells often require additional factors that encourage their attachment to a solid support, such as laminins, type I and type II collagens, chondroitin sulfate, fibronectin, "superfibronectin" and fibronectin-like polymers, gelatin, poly-D and poly-L-lysine, thrombospondin and vitronectin. Other suitable coating materials, based on or composed of basal membrane-derived protein mixtures, can be used. Some of these are commercially available, such as Matrigel. Coatings may use extracellular matrix proteins, and suitable derivatives thereof, as well as mixtures of these proteins. One embodiment of the present invention utilizes fibronectin. See, for example, Ohashi et al., Nature Medicine, 13:880-885 (2007); Matsumoto et al., J Bioscience and Bioengineering, 105:350-354 (2008); Kirouac et al., Cell Stem Cell, 3:369-381 (2008); Chua et al., Biomaterials, 26:2537-2547 (2005); Drobinskaya et al., Stem Cells, 26:2245-2256 (2008); Dvir- Ginzberg et al., FASEB J, 22:1440-1449 (2008); Turner et al., JBiomed Mater Res Part B: Appl Biomater, 82B:156-168 (2007); and Miyazawa et al., Journal of Gastroenterology and Hepatology, 22:1959-1964 (2007)).

Once established in culture, cells can be used fresh or frozen and stored as frozen stocks, using, for example, DMEM with 20%-40% PCS and 10% DMSO. In one embodiment, 20% PCS is used. Other methods for preparing frozen stocks for cultured cells are also available to those of skill in the art.

For the purposes of this application, the additional culture methods as well as the other culture methods also apply to the bioreactor methods, with respect to the medium components and conditions described above. As an example, in an exemplified embodiment, the oxygen concentration is 5%, serum is about 19% and both EGF and PDGF are added to the medium.

### Pharmaceutical Formulations

U.S. 7,015,037 teaches pharmaceutical formulations. In certain embodiments, the cell populations are present within a composition adapted for and suitable for delivery, i.e., physiologically compatible.

In some embodiments the purity of the cells (or conditioned medium) for administration to a subject is about 100% (substantially homogeneous). In other embodiments it is 95% to 100%. In some embodiments it is 85% to 95%. Particularly, in the case of admixtures with other cells, the percentage can be about 10%-15%, 15%-20%, 20%-25%, 25%-30%, 30%-35%, 35%-40%, 40%-45%, 45%-50%, 60%- 70%, 70%-80%, 80%-90%, or 90%-95%. Or isolation/purity can be expressed in terms of cell doublings where the cells have undergone, for example, 10-20, 20-30, 30-40, 40-50 or more cell doublings.

The choice of formulation for administering the cells for a given application will depend on a variety of factors. Prominent among these will be the species of subject, the nature of the condition being treated, its state and distribution in the subject, the nature of other therapies and agents that are being administered, the optimum route for administration, survivability via the route, the dosing regimen, and other factors that will be apparent to those skilled in the art. For instance, the choice of suitable carriers and other additives will depend on the exact route of administration and the nature of the particular dosage form.

Final formulations of the aqueous suspension of cells/medium will typically involve adjusting the ionic strength of the suspension to isotonicity (i.e., about 0.1 to 0.2) and to physiological pH (i.e., about pH 6.8 to 7.5). The final formulation will also typically contain a fluid lubricant.

In some embodiments, cells/medium are formulated in a unit dosage injectable form, such as a solution, suspension, or emulsion. Pharmaceutical formulations suitable for injection of cells/medium typically are sterile aqueous solutions and dispersions. Carriers for injectable formulations can be a solvent or dispersing medium containing, for example, water, saline, phosphate buffered saline, polyol (for example, glycerol, propylene glycol, liquid polyethylene glycol, and the like), and suitable mixtures thereof.

The skilled artisan can readily determine the amount of cells and optional additives, vehicles, and/or carrier in compositions to be administered in methods of the invention. Typically, any additives (in addition to the cells) are present in an amount of 0.001 to 50 wt % in solution, such as in phosphate buffered saline. The active ingredient is present in the order of micrograms to milligrams, such as about 0.0001 to about 5 wt %, preferably about 0.0001 to about 1 wt %, most preferably about 0.0001 to about 0.05 wt % or about 0.001 to about 20 wt %, preferably about 0.01 to about 10 wt %, and most preferably about 0.05 to about 5 wt %.

### EXAMPLES

### Example 1. Expanding MultiStem From a Cell Bank By Means Of A Hollow Fiber Bioreactor Cell Expansion System

A cell expansion system, essentially as described in U.S. 2008/0220523 and Antwiler et al., was used to expand an input population of MultiStem, which is a substantially homogeneous population. The system was designated Quantum CES. It provided a bench-top expansion system that is particularly useful for autologous expansion. (But it is also useful for allogeneic expansion.) Based on the results shown in this application, greater than 10⁸ cells can be obtained from a single donor.

Multi-Stem was thawed after having been stored in a frozen cell bank. For two experiments, 10 x 10⁶ MultiStem were inoculated into a continuous flow bioreactor with an internal fluid volume of 184 ml and an external fluid volume of 303 ml, containing 1.1 x 10⁴ hollow fibers with a total surface area (intracapillary) of approximately 2.1 m² (3255in²), a wall thickness of 50 µm, inner diameter of 215 µm, and total length of 295 mm (average 30 mm per fiber, range of 10-50 mm). Pore size had an approximate cutoff of 16 kDa. The cells adhered to the inside of the hollow fibers. Cells were cultured for six days. 752 x 10⁶, 753 x 10⁶ and 782 x 10⁶ cells were produced in three independent experiments starting from the same seeding stock.

The flow rate could be variable but generally increased as the cells proliferated so that the nutrients were provided in sufficient amounts and the waste products were removed in sufficient amounts. In general, the flow rate can be increased around ten times. However, this parameter was adjusted by monitoring the waste products, such as lactate and glucose levels, in the medium.

The wall material was a composite of PA/PAES/PVP.

Also, in this particular embodiment, the fibers were coated with fibronectin.

For established cells, that is those that were derived from a previously isolated, expanded, substantially purified culture, 5 mg of fibronectin is used and the coating is done overnight. Then the coating is washed out and replaced by medium. After seeding, the cells are attached for 24 hours with no flow through the intracellular loop. After that the feed rate is started at about 0.1 ml per minute. Based on the lactate level and on the doubling rates of the cells, the feed rate is doubled approximately every 24 hours. In the first three days the inventors attempted to keep the lactate level around 0.5 g/1. The last three days it is kept at around 1 g/1 with a maximum of 1.5 g/1. On the last day (i.e. when the cells are expanded to their maximum) the feed rate is about 3 ml/minute.

Cells are harvested using about 200 ml 2.5% trypsin with a 2.5 minute incubation period and a harvest volume of about 500 ml.

When a bone marrow aspirate is used, the intracapillary surface is coated with about 10 mg of fibronectin with a 48 hour attachment phase. The feed rate is adjusted so that if the lactate level goes up to 0.4 to 0.5 g/1, the feed rate is doubled. The rate generally is not as high compared to a culture starting from cells that have been established. In one experiment, about 25-30 ml of bone marrow aspirate was used to produce about 4 x 10⁶ cells from total BMMC. For that, bone marrow was subjected to a ficoll gradient to remove non-nucleated cells.

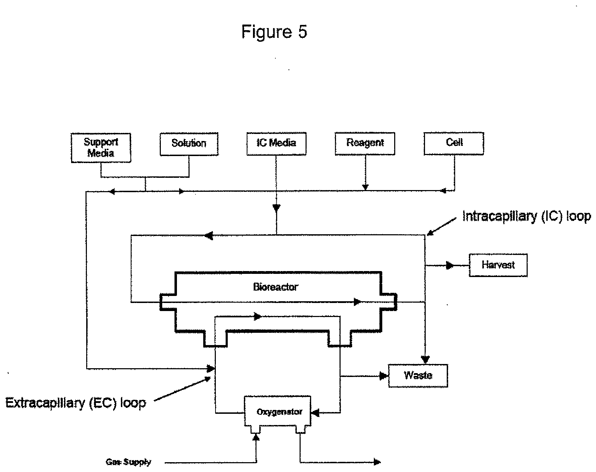

Details are found in Figure 5. The flow scheme is comprised of 2 loops: the intracapillary (IC) and extracapillary (EC) loop. The IC loop has 3 inlet lines: cell line, reagent line and IC media line. Bags are attached to these lines for adding cells, coating agent, trypsin and media. Each line is controlled by a valve, which has 2 positions, open or close. The inlet rate is controlled by the IC inlet pump (1). Circulation within the system is controlled by the IC circulation pump (2). The IC circuit contains 150 ml. The EC loop has 2 inlet lines: Wash (solution) and EC media line. Bags are attached to these lines for washing with PBS and adding medium to the EC loop. The EC inlet rate is controlled by the EC inlet pump (3) and the circulation within this circuit by the EC circulation pump (4). In the EC loop an oxygenator is present. This oxygenator is attached to an external gas cylinder (for MultiStem this is 90% N2; 5% O2; 5% CO2) and maintains the gas concentrations for the EC medium. The IC medium is brought to equilibrium because of perfusion of the gases through the membrane in the bioreactor. The EC circuit contains around 250 ml. There are 2 ways for solutions/cells to leave the system: in the waste bag or the harvest bag. The IC as well as the EC loop have a connection to the waste bag which is controlled by a valve. The harvest bag only as a connection with the IC loop and is also controlled by a valve.

The input population and the output population were compared. Specifically, the input population was assessed for gene expression of certain selected genes and this was compared with the output population. In addition, morphology and ploidy were compared. The output population was substantially identical to the input population with respect to the measured parameters.

### Example 2, Producing MultiStem From Bone Marrow in a Hollow Fiber Bioreactor Cell Expansion System

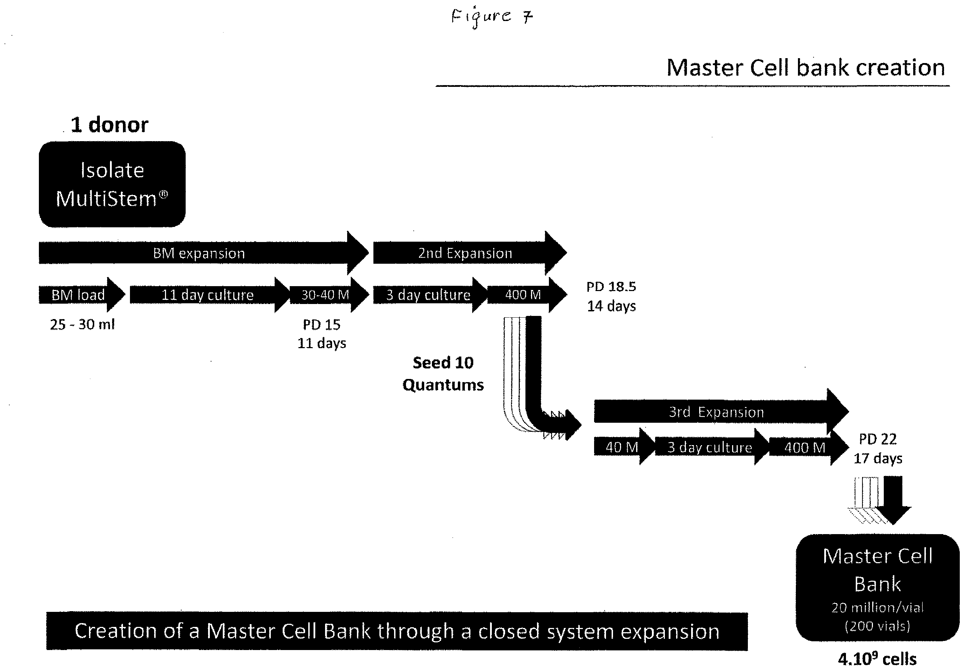

The cell expansion system described above was used to produce MultiStem from bone marrow mononuclear cells. The expansion results are shown in Figure 6. In one possible regimen, starting from a bone marrow aspirate, it is possible to create a MultiStem master cell bank within 17 days. This takes one run from bone marrow to 15 population doublings and an expansion run to this harvest to reach a population doubling of 18.5 and an average of ten runs to create a master cell bank (200 vials of 20 million cells). These 200 vials can then be used to create working cell banks. The schematic is shown in Figure 7. However, the master cell bank could be increased or decreased, such as up to 10-fold more cells or down to 10-fold less cells.

The output cells, following expansion, had the immunomodulatory properties associated with MultiStem and expressed telomerase.

## Claims

1. A method of expanding cells *ex vivo,* the method comprising:
a) seeding the cells on a hollow fiber substrate so that the cells adhere to the substrate;
b) expanding the adhered cells on the substrate; and
c) removing the expanded cells from the substrate;
wherein the cells are non-embryonic stem, non-germ cells; express telomerase, are not transformed, have a normal karyotype, and can differentiate into at least one cell type of at least two of the
endodermal, ectodermal, and mesodermal embryonic lineages.

2. The method of claim 1 further comprising:
d) reseeding the removed cells on the same or different substrate; and
e) repeating steps b) - d) until a desired number of expanded cells is reached.

3. The method of claim 1 in which the hollow fiber substrate is in a closed continuous perfusion bioreactor.

4. The method of claim 2 in which the cells are expanded about 10-100 fold.

5. The method of any previous claim wherein the hollow fiber is coated.

6. The method of claim 5 wherein the coating is fibronectin.

7. The method of claim 6 wherein the hollow fiber wall material is a blend of PA/PAES/PVP.

8. The method of claims 1-7 wherein the cells in step (a) comprise a substantially homogeneous population.

9. The method of claims 1-8 wherein step (a) comprises seeding cells from a tissue, the tissue selected from bone marrow, umbilical cord blood, umbilical cord matrix, peripheral blood, placenta, placental blood, muscle, brain, kidney, or other solid organs.

10. The method of any previous claim wherein the non-embryonic stem, non-germ cells further express one or more of oct4, rex-1, rox-1, or sox-2.

## Patentansprüche

1. Verfahren zum Expandieren von Zellen *ex vivo,* das Verfahren umfassend:
a) Aussäen der Zellen auf ein Hohlfasersubstrat, so dass die Zellen an dem Substrat haften;
b) Expandieren der anhaftenden Zellen auf dem Substrat; und
c) Entfernen der expandierten Zellen von dem Substrat;
wobei die Zellen nicht-embryonale Stamm, Nicht-Keimzellen sind, Telomerase exprimieren, nicht transformiert sind, einen normalen Karotyp aufweisen, und in mindestens einen Zelltyp von mindestens zwei der endodermalen, ektodermalen und mesodermalen embryonalen Abstammungen differenzieren können.

2. Verfahren nach Anspruch 1, ferner umfassend
d) erneutes Aussäen der entfernten Zellen auf dem gleichen oder einem anderen Substrat; und
e) Wiederholen der Schritte b) bis d), bis eine gewünschte Anzahl von expandierten Zellen erreicht ist.

3. Verfahren nach Anspruch 1, bei dem sich das Hohlfasersubstrat in einem geschlossenen kontinuierlichen Perfusionsbioreaktor befindet.

4. Verfahren nach Anspruch 2, bei dem die Zellen ungefähr 10-100fach expandiert werden.

5. Verfahren nach einem der vorhergehenden Ansprüche, wobei die Hohlfaser beschichtet ist.

6. Verfahren nach Anspruch 5, wobei die Beschichtung Fibronektin ist.

7. Verfahren nach Anspruch 6, wobei das Hohlfaserwandmaterial eine Mischung aus PA/PAES/PVP ist.

8. Verfahren nach den Ansprüchen 1 bis 7, wobei die Zellen in Schritt (a) eine im wesentlichen homogene Population umfassen.

9. Verfahren nach Ansprüchen 1 bis 8, wobei Schritt (a) Säen von Zellen aus einem Gewebe umfasst, wobei das Gewebe ausgewählt ist aus Knochenmark, Nabelschnurblut, Nabelschnurmatrix, peripherem Blut, Plazenta, Plazentablut, Muskel, Gehirn, Niere oder anderen festen Organen.

10. Verfahren nach einem der vorhergehenden Ansprüche, wobei die nichtembryonalen Stamm, Nicht-Keimzellen ferner eines oder mehrere von oct4, rex-1, rox-1 oder sox-2 exprimieren.

## Revendications

1. Procédé d'expansion de cellules *ex vivo,* le procédé comprenant :
a) l'ensemencement des cellules sur un substrat en fibres creuses de manière que les cellules adhèrent au substrat ;
b) l'expansion des cellules collées sur le substrat ; et
c) le retrait des cellules expansées du substrat ;
les cellules étant des cellules non germinales non embryonnaires ; exprimant la télomérase, n'étant pas transformées, présentant un caryotype normal et pouvant se différencier en au moins un type de cellule d'au moins deux des lignées embryonnaires endodermiques, ectodermiques et mésodermiques.

2. Procédé selon la revendication 1 comprenant en outre :
d) le réensemencement des cellules retirées sur le même substrat ou un substrat différent ; et
e) la répétition des étapes b) à d) jusqu'à ce qu'un nombre désiré de cellules expansées soit atteint.

3. Procédé selon la revendication 1 dans lequel le substrat en fibres creuses se trouve dans un bioréacteur fermé à perfusion en continu.

4. Procédé selon la revendication 2 dans lequel les cellules sont expansées d'environ 10 à 100 fois.

5. Procédé selon l'une quelconque des revendications précédentes dans lequel la fibre creuse est recouverte.

6. Procédé selon la revendication 5 dans lequel le revêtement est la fibronectine.

7. Procédé selon la revendication 6 dans lequel le matériau de paroi en fibres creuses est un mélange de PA/PAES/PVP.

8. Procédé selon les revendications 1 à 7 dans lequel les cellules dans l'étape (a) comprennent une population sensiblement homogène.

9. Procédé selon les revendications 1 à 8 dans lequel l'étape (a) comprend l'ensemencement de cellules provenant d'un tissu, le tissu étant choisi parmi la moelle épinière, le sang de cordon ombilical, la matrice de cordon ombilical, le sang périphérique, le placenta, le sang de placenta, le muscle, le cerveau, le rein ou d'autres organes solides.

10. Procédé selon l'une quelconque des revendications précédentes dans lequel les cellules non germinales, non embryonnaires expriment en outre au moins un des éléments parmi oct4, rex-1, rox-1 ou sox-2.
